# EUROPÄISCHE PATENTSCHRIFT

(11) **EP 0 578 054 B1**
(45) Veröffentlichungstag und Bekanntmachung des Hinweises auf die Patenterteilung: **19.05.1999**
(21) Anmeldenummer: 93110020.0
(22) Anmeldetag: 23.06.1993
(51) Int. Cl.: C07D 239/26, C09K 19/34, C07D 241/12, C07D 237/08, C07D 213/30, C07D 285/12, C07D 405/12, C07C 43/205, C09K 19/12, G02F 1/13

(54) **Meta-substituierte Sechsringaromaten zur Verwendung in Flüssigkristallmischungen**
Meta-substituted six membered aromatic rings for use in liquid crystal mixtures
Cyces aromatiques a six membres substitués en méta pour l'utilisation dans des mélanges de cristaux liquides

(30) Priorität: 09.07.1992 DE 4222565
(43) Veröffentlichungstag der Anmeldung: 12.01.1994
(73) Patentinhaber: HOECHST AKTIENGESELLSCHAFT, 65926 Frankfurt am Main (DE)
(72) Erfinder: Jungbauer, Dietmar, Dr., D-64331 Weiterstadt (DE); Schlosser, Hubert, Dr., D-6246 Glashütten/Ts (DE)
(74) Vertreter: Bardehle, Heinz, Dipl.-Ing.

(56) Entgegenhaltungen:
- WO-A-86/05484
- FR-A- 1 488 494
- JP-A- 2 255 635
- DATABASE WPIL Section Ch, Week 9047, Derwent Publications Ltd., London, GB; Class E07, AN 90-352788

## Beschreibung

Flüssigkristalle haben insbesondere im letzten Jahrzehnt Eingang in verschiedene technische Gebiete gefunden, in denen elektrooptische und Anzeigevorrichtungs-Eigenschaften gefragt sind (z. B. in Uhren, Taschenrechner-und Schreibmaschinenanzeigen). Diese Anzeigevorrichtungen beruhen auf den dielektrischen Ausrichtungseffekten in den nematischen, cholesterischen und smektischen Phasen der flüssigkristallinen Verbindungen, wobei - verursacht durch die dielektrische Anisotropie - die molekulare Längsachse der Verbindungen eine bevorzugte Ausrichtung in einem angelegten elektrischen Feld einnimmt. Die üblichen Schaltzeiten bei diesen Anzeigevorrichtungen sind für viele andere potentielle Anwendungsgebiete von Flüssigkristallen zu lang. Dieser Nachteil macht sich insbesondere dann bemerkbar, wenn eine große Anzahl von Bildpunkten angesteuert werden muß. Die Herstellungskosten von Geräten, die größere Bildschirmflächen enthalten, sind dann im allgemeinen zu hoch.

Neben den nematischen und cholesterischen Flüssigkristallen haben seit einigen wenigen Jahren in zunehmenden Maße auch optisch aktive smektische Flüssigkristalle an Bedeutung gewonnen.

Clark und Lagerwall konnten zeigen, daß die Verwendung ferroelektrischer Flüssigkristalle (FLC) in sehr dünnen Zellen zu elektrooptischen Schalt-oder Anzeigeelementen führt, die im Vergleich zu den herkömmlichen TN ("twisted nematic")-Zellen um bis zu einem Faktor 1000 schnellere Schaltzeiten haben (vgl. z. B. Lagerwall et al. "Ferroelectric Liquid Crystals for Displays", SID Symposium, October Meeting 1985, San Diego, Ca., USA). Aufgrund dieser und anderer günstiger Eigenschaften z. B. der bistabilen Schaltmöglichkeit und des nahezu blickwinkelunabhängigen Kontrasts, sind FLCs grundsätzlich für die obengenannten Anwendungsgebiete. z. B. über eine Matrixansteuerung, gut geeignet. Auch auf dem Gebiet der räumlichen Lichtmodulatoren (vgl. z. B. U. Efron in "Spatial Light Modulators and Applications", SPIE Vol. 1150, S. 46 ff) sind ferroelektrische Flüssigkristalle wegen ihres hohen Kontrasts und ihrer hohen Schaltgeschwindigkeit besonders geeignet. Die Schaltgeschwindigkeit ferroelektrischer Flüssigkristallmischungen reicht im allgemeinen aber noch nicht aus, um z. B. hochauflösende, schnelle Anzeigeelemente zu betreiben. Daher ist es wünschenswert, Komponenten aufzufinden, die die Schaltgeschwindigkeit flüssigkristalliner Mischungen steigern.

Die Molekülstruktur der für obengenannte Anwendungsgebiete einsetzbaren Flüssigkristalle baut im wesentlichen auf para- bzw. 1,4-substituierte aromatische und gesättigte Sechsringe, wie 1,4-Phenylen, Pyridin-2,5-diyl, Pyrimidin-2,5-diyl und trans 1,4-Cyclohexylen auf, die durch Verknüpfung untereinander, entweder direkt oder über geeignete Zwischenstücke, sowie durch Verknüpfung mit geeigneten terminalen Gruppen die aus zahlreichen Beispielen bekannten (siehe z. B. in: D. Demus, H. Zaschke, "Flüssige Kristalle in Tabellen I und II, VEB Deutscher Verlag für Grundstoffindustrie, Leipzig 1974 und 1984) langgestreckten "stäbchenförmigen" Moleküle liefern.

Flüssigkristalline Verbindungen, die als Substruktur einen ausschließlich meta- bzw. 1,3-substituierten Sechsringaromaten enthalten, sind dagegen bis jetzt nur in JP-A-2 255 635.

Die beschriebenen Verbindungen sollen die Schaltgeschwindigkeiten und das Tieftemperaturverhalten von Flüssigkristallmischungen verbessern. Als Substituent wird Trifluormethyl eingesetzt.

Aufgabe der vorliegenden Erfindung ist die Bereitstellung von flüssigkristallinen Verbindungen, deren Zugabe zu Flüssigkristallmischungen zu einer Verbesserung des optischen Schaltwinkels und zur Reduktion der kritischen Pulsfläche beim elektrooptischen Schalten führt.

Gegenstand der vorliegenden Erfindung ist daher die Verwendung von Verbindungen der Formel (I) 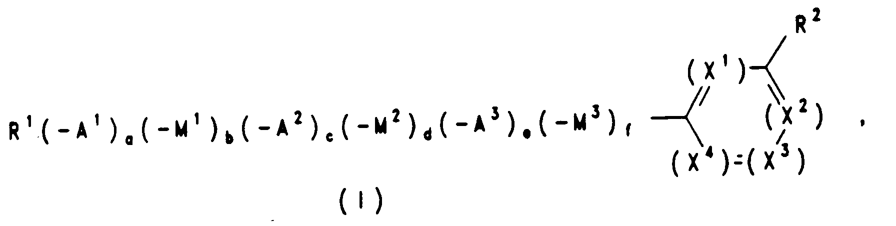 in der die Symbole und Indizes folgende Bedeutung haben:
- R¹, R²: sind unabhängig voneinander ein geradkettiger oder verzweigter Alkylrest mit 1 bis 22 C-Atomen (mit oder ohne asymmetrische C-Atome), wobei auch eine oder zwei -CH₂-Gruppen durch -O-, -S-, -CO-, -CO-O-, -O-CO-, -CO-S-, S-CO-, -O-CO-O-, -CH=CH-, -C≡C-, Cyclopropyldiyl oder -Si(CH₃)₂- ersetzt sein können und wobei auch ein oder mehrere H-Atome des Alkylrestes durch F, Cl, Br oder CN substituiert sein können, oder eine der nachfolgenden chiralen Gruppen: 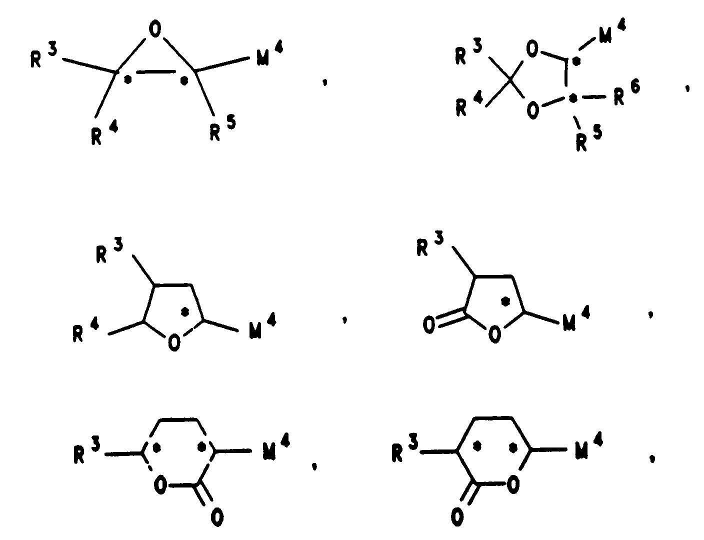 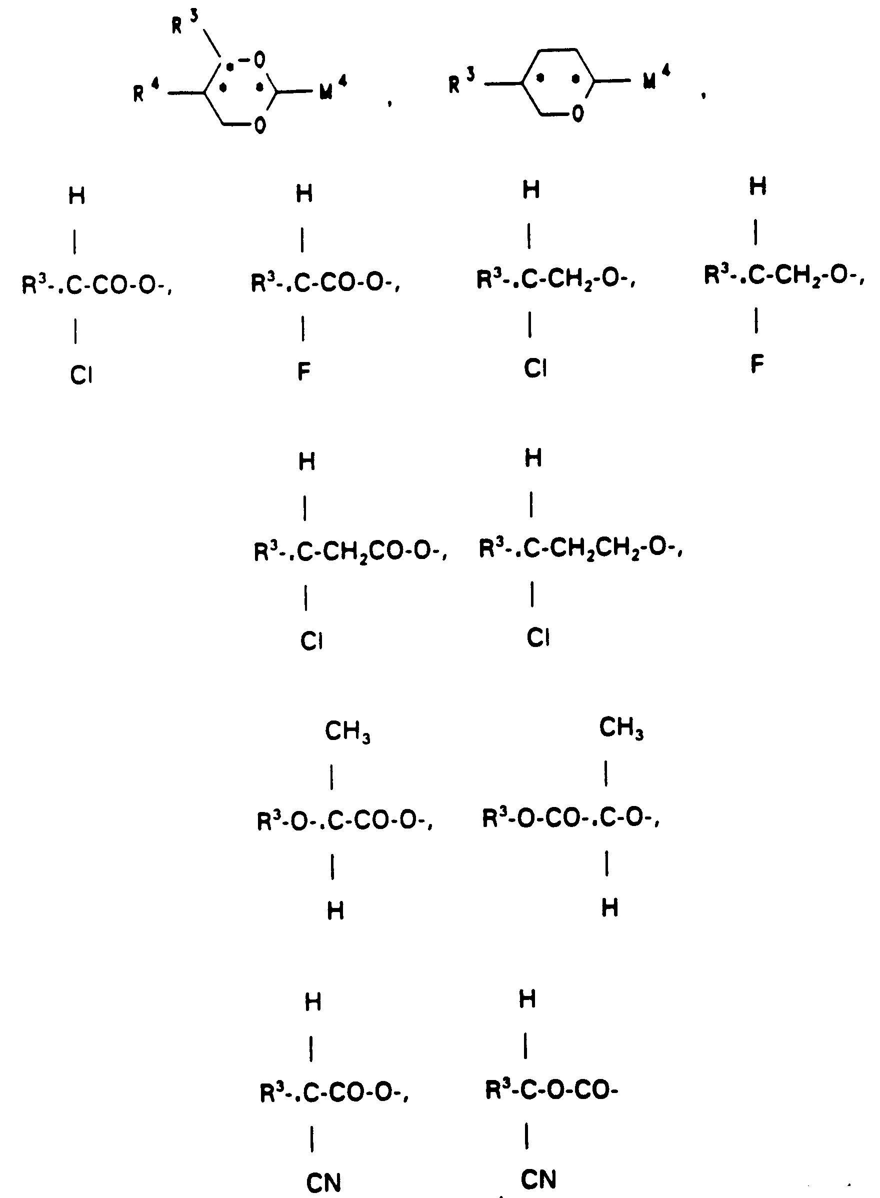 in Flüssigkristallmischungen zur Verbesserung des optischen Schaltwinkels und zur Reduktion der kritischen Pulsfläche beim elektrooptischen Schalten. Auch ein Teil dieser Verbindungen ist Gegenstand der vorliegenden Erfindung.

Bevorzugt sind R¹, R² unabhängig voneinander ein geradkettiger oder verzweigter Alkylrest mit 1 bis 15 C-Atomen (mit oder ohne asymmetrische C-Atome), wobei auch eine oder zwei nicht benachbarte -CH₂-Gruppen durch -O-, -S-, -CO-, -CO-O-, -O-CO-, -OCO-O-, -CH=CH-, -C≡C-, Cyclopropyldiyl oder -Si(CH₃)₂- ersetzt sein können, oder eine der nachfolgenden chiralen Gruppen:

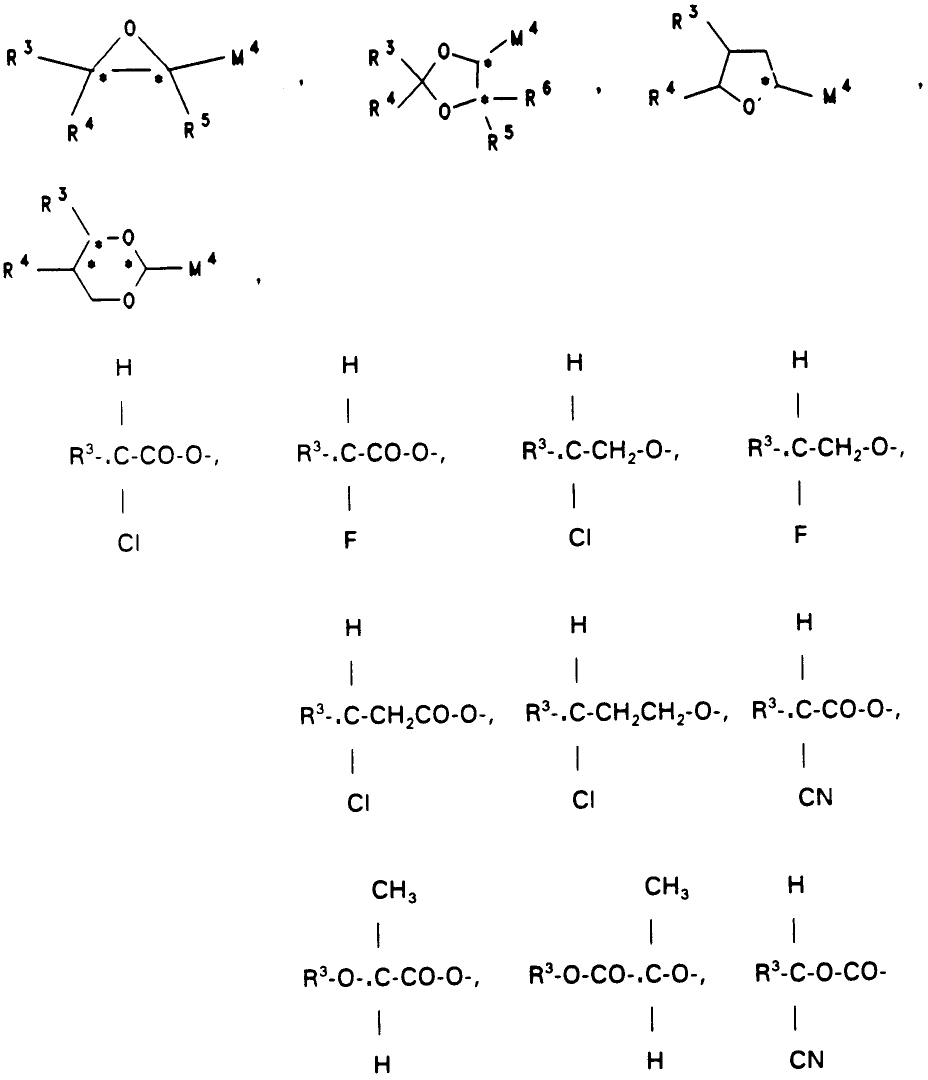

Besonders bevorzugt sind R¹, R² unabhängig voneinander ein geradkettiger oder verzweigter Alkylrest mit 1 bis 10 C-Atomen (mit oder ohne asymmetrische C-Atome), wobei auch eine oder zwei -CH₂-Gruppen durch -O-, -CO-, -CO-O-, -O-CO-, -CH=CH-, -C≡C-, Cyclopropyldiyl oder -Si(CH₃)₂- ersetzt sein können, oder eine der nachfolgenden chiralen Gruppen:

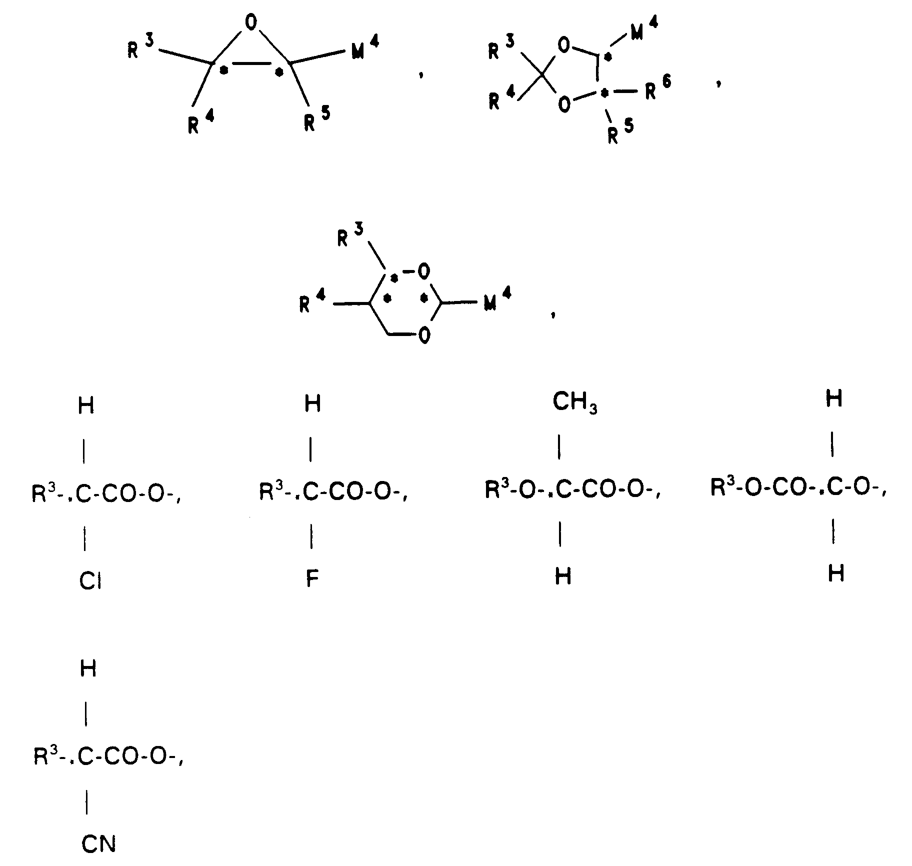

Insbesondere bevorzugt sind R¹, R² unabhängig voneinander ein Alkylrest mit 1 bis 8 C-Atomen, wobei auch eine oder zwei -CH₂-Gruppen durch -O-, -OCO-oder Cyclopropyldiyl ersetzt sein können, oder die chiralen Gruppen:

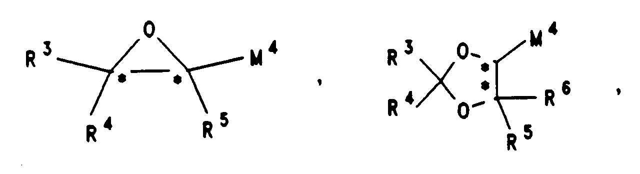

R³, R⁴, R⁵, R⁶ sind unabhängig voneinander H oder ein geradkettiger oder verzweigter Alkylrest mit 1 bis 22 C-Atomen, wobei auch eine oder zwei CH₂-Gruppen durch -O-, -S-, -CO-, -CO-O-, -O-CO-, -CO-S-, -S-CO-, -O-CO-O-, -CH=CH-, -C≡C-, Cyclopropyldiyl oder -Si(CH₃)₂- ersetzt sein können, oder R³ und R⁴ können zusammen auch -(CH₂)₄- oder -(CH₂)₅- sein, wenn sie als Substituenten an ein Dioxolan-System gebunden sind.

Bevorzugt sind R³, R⁴, R⁵, R⁶ unabhängig voneinander H oder ein geradkettiger oder verzweigter Alkylrest mit 1 bis 15 C-Atomen, wobei auch eine oder zwei -CH₂-Gruppen durch -O-, -S-, -CO-, -CO-O-, -O-CO-, -CO-S-, -S-CO-, -O-CO-O-, -CH=CH-, -C≡C-, Cyclopropyldiyl oder -Si(CH₃)₂- ersetzt sein können, oder R³ und R⁴ können zusammen auch -(CH₂)₄- oder -(CH₂)₅- sein, wenn sie als Substituenten an ein Dioxolan-System gebunden sind;

Besonders bevorzugt sind R³, R⁴, R⁵, R⁶ unabhängig voneinander H oder ein geradkettiger oder verzweigter Alkylrest mit 1 bis 10 C-Atomen, wobei auch eine oder zwei -CH₂-Gruppen durch -O-, -S-, -CO-, -CO-O-, -O-CO-, -CO-S-, -S-CO-, -O-CO-O-, -CH=CH-, -C≡C-, Cyclopropyldiyl oder -Si(CH₃)₂- ersetzt sein können, oder R³ und R⁴ können zusammen auch -(CH₂)₄- oder -(CH₂)₅- sein, wenn sie als Substituenten an ein Dioxolan-System gebunden sind;

Insbesondere sind R³, R⁴, R⁵, R⁶ unabhängig voneinander H oder ein geradkettiger oder verzweigter Alkylrest mit 1 bis 8 C-Atomen;

A¹, A², A³ sind unabhängig voneinander 1,4-Phenylen, wobei ein oder zwei H-Atome durch F, Cl und/oder CN ersetzt sein können, Pyrazin-2,5-diyl, Pyridazin-3,6-diyl, Pyridin-2,5-diyl, Pyrimidin-2,5-diyl, bei denen ein oder zwei H-Atome durch F ersetzt sein können, trans-1,4-Cyclohexylen, bei dem ein oder zwei H-Atome durch -CN und/oder -CH₃ ersetzt sein können, (1,3,4)-Thiadiazol-2,5-diyl, 1,3-Dioxan-2,5-diyl, 1,3-Dithian-2,5-diyl, 1,3-Thiazol-2,4-diyl, 1,3-Thiazol-2,5-diyl, Thiophen-2,4-diyl, Thiophen-2,5-diyl, Piperazin-1,4-diyl, Piperazin-2,5-diyl, Naphthalin-2,6-diyl, Bicyclo[2.2.2]octan-1,4-diyl, 1,3-Dioxaborinan-2,5-diyl oder trans-Dekalin-2,6-diyl;

A¹ kann auch eine Gruppe der Formel 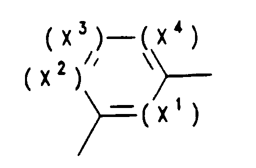 sein;

Bevorzugt sind A¹, A², A³ unabhängig voneinander 1,4-Phenylen, Pyrazin-2,5-diyl, Pyridazin-3,6-diyl, Pyridin-2,5-diyl, Pyrimidin-2,5-diyl, bei denen ein oder zwei H-Atome durch F ersetzt sein können, trans-1,4-Cyclohexylen, (1,3,4)-Thiadiazol-2,5-diyl, 1,3-Dioxan-2,5-diyl, Naphthalin-2,6-diyl oder Bicyclo[2.2.2]octan-1,4-diyl;

A¹ kann auch eine Gruppe der Formel 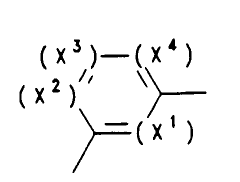 sein;

Besonders bevorzugt sind A¹, A², A³ unabhängig voneinander 1,4-Phenylen, Pyrazin-2,5-diyl, Pyridin-2,5-diyl, Pyrimidin-2,5-diyl, bei denen ein oder zwei H-Atome durch F ersetzt sein können, trans-1,4-Cyclohexylen, (1,3,4)-Thiadiazol-2,5-diyl, Naphthalin-2,6-diyl oder 1,3-Dioxan-2,5-diyl,

A¹ kann auch eine Gruppe der Formel 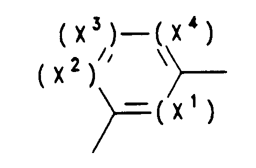 sein;

Insbesondere bevorzugt sind A¹, A², A³ unabhängig voneinander 1,4-Phenylen, Pyridin-2,5-diyl, Pyrimidin-2,5-diyl, bei denen ein oder zwei H-Atome durch F ersetzt sein können oder trans-1,4-Cyclohexylen;

A¹ kann auch eine Gruppe der Formel 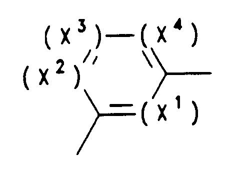 sein;

M¹, M², M³ sind unabhängig voneinander -O-, -S-, -CO-, -CO-O-, -O-CO-, -CO-S-, -S-CO-, -O-CO-O-, -CH₂-O-, -O-CH₂-, -CH₂CH₂-, -CH=CH- oder -C≡C-;

Vorzugsweise sind M¹, M², M³ unabhängig voneinander -O-, -CO-, -CO-O-, -O-CO-, -CH₂-O-, -O-CH₂-, -CH₂CH₂-, -CH=CH- oder -C≡C-;

Besonders bevorzugt sind M¹, M², M³ unabhängig voneinander -O-, -CO-O-, -O-CO-, -CH₂-O-, -O-CH₂-, -CH₂CH₂-, -CH=CH- oder -C≡C-;

Insbesondere bevorzugt sind M¹, M², M³ unabhängig voneinander -O-, -CO-O-, -O-CO-, -O-CH₂-, -CH₂-O- oder -CH₂CH₂-;

M⁴ ist -CH₂-O-, -O-CH₂-, -CO-O-, -O-CO- oder eine Einfachbindung;

X¹, X², X³, X⁴ sind CH, CF oder N, wobei die Zahl der N-Atome 0, 1 oder 2 beträgt;

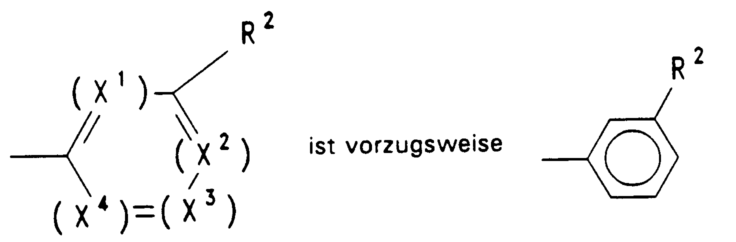

ist vorzugsweise a, b, c, d, e, f sind Null oder Eins, unter der Bedingung, daß die Summe a + c + e 1, 2 oder 3 ist.

* ist ein chirales Zentrum.

Die Herstellung der erfindungsgemäßen Verbindungen erfolgt nach an sich literaturbekannten Methoden, wie sie in Standardwerken zur Organischen Synthese, z. B. Houben-Weyl, Methoden der Organischen Chemie, Georg-Thieme-Verlag, Stuttgart, beschrieben werden.

Die Herstellung erfolgt dabei unter Reaktionsbedingungen, die für die genannten Umsetzungen bekannt und geeignet sind. Dabei kann man auch von an sich bekannten, hier nicht näher erwähnten Varianten Gebrauch machen. Was die Synthese des Restes "RAMAMAM" betrifft, so sei beispielsweise verwiesen auf DE-OS 23 44 732, 24 50 088, 24 29 093, 25 02 904, 26 36 684, 27 01 591 und 27 52 975 für Verbindungen mit 1,4-Cyclohexylen und 1,4-Phenylen-Gruppen; DE-PS 26 41 724 für Verbindungen mit Pyrimidin-2,5-diyl-Gruppen; DE-OS 40 26 223 und EP-OS 03 91 203 für Verbindungen mit Pyridin-2,5-diyl-Gruppen; DE-OS 32 31 462 für Verbindungen mit Pyridazin-3,6-diyl-Gruppen; DE-OS 29 44 905 und 32 27 916 für Verbindungen mit 1,3-Dioxan-2,5-diyl-Gruppen; DD 160 061 für Verbindungen mit 1,3-Dithian-2,5-diyl-Gruppen; US 4,261,652 und 4,219,256 für Verbindungen mit 1,4-Bicyclo-[2.2.2]octan-1,4-diyl-Gruppen; N. Miyaura, T. Yanagi und A. Suzuki in Synthetic Communications 11 (1981), S.513-519 für die direkte Verknüpfung von Aromaten und Heteroaromaten;
DE-OS 32 01 721 für Verbindungen mit -CH₂CH₂-Brückengliedern und Koji Seto ei al. in Liquid Crystals 8 1990), S. 861-870 für Verbindungen mit -C≡C-Brückengliedern.

Strategien zur Synthese 1,3-disubstituierter Benzole finden sich beispielsweise in J. March, Advanced Organic Chemistry, McGraw-Hill Kogaskusha Ltd..

Die Darstellung 2,4-, 2,6- und 3,5-disubstituierter Pyridine, 2,6-disubstituierter Pyrazine, 2,4- und 4,6-disustituierter Pyrimidine und 3,5-disubstituierter Pyridazine findet sich beispielsweise in den entsprechenden Bänden der Serie "The Chemistry of Heterocyclic Compounds" von A. Weissberger und E.C. Taylor (Herausgeber).

Die Ausgangsstoffe können gewünschtenfalls auch in situ gebildet werden, und zwar derart, daß man sie aus dem Reaktionsgemisch nicht isoliert, sondern sofort weiter zu den Verbindungen der Formel (I) umsetzt.

Die erfindungsgemäßen Verbindungen der allgemeinen Formel (I) sind als Komponenten zur Verwendung für Flüssigkristallmischungen, insbesondere für ferroelektrische Flüssigkristallmischungen, geeignet, wobei die Verbindungen der allgemeinen Formel (I) nicht notwendigerweise selbst flüssigkristallin sein müssen. Dabei können die LC-Mischungen 0,01 bis 60 Gew.-%, bevorzugt 0,1 bis 40 Gew.-%, besonders bevorzugt 0,1 bis 20 Gew.-%, an einer oder mehreren der Verbindungen nach Formel (I) enthalten. Die anderen Bestandteile werden vorzugsweise ausgewählt aus bekannten Verbindungen mit nematischen, cholesterischen und/oder smektischen Phasen; dazu gehören beispielsweise Schiffsche Basen, Biphenyle, Terphenyle, Phenylcyclohexane, Cyclohexylbiphenyle, N, S oder O-haltige Heterocyclen, z. B. Pyrimidine, Zimtsäureester, Cholesterinester oder verschieden überbrückte, terminalpolar mehrkernige Ester von p-Alkylbenzoesäuren.
Solche Mischungen enthalten vorzugsweise 2 bis 20 Komponenten.

Die erfindungsgemäßen Verbindungen können auch in nematischen Flüssigkristallmischungen eingesetzt werden.

Es zeigte sich nun überraschenderweise, daß durch Zugabe von Verbindungen der allgemeinen Formel (I) der optische Schaltwinkel verbessert und die kritische Pulsfläche (critical pulse area = CPA) beim elektrooptischen Schalten dieser Flüssigkristallmischungen erheblich reduziert werden kann.

Die Verbindungen der Formel I besitzen darüberhinaus einen breiten Anwendungsbereich. In Abhängigkeit von der Auswahl der Substituenten können diese Verbindungen als Basismaterialien dienen, aus denen flüssigkristalline smektische, insbesondere ferroelektrische Phasen zum überwiegenden Teil zusammengesetzt sind; es können aber auch Verbindungen der Formel (I) flüssigkristallinen Basismaterialien aus anderen Verbindungsklassen zugesetzt werden, um beispielsweise die dielektrische und/oder optische Anisotropie und/oder die Viskosität und/oder die spontane Polarisation und/oder die Phasenbereiche und/oder den Tiltwinkel und/oder den Pitch eines solchen Dielektrikums zu variieren.

Die erfindungsgemäßen Mischungen wiederum können Anwendung finden in elektrooptischen oder vollständig optischen Elementen, z. B. Anzeigeelementen, Schaltelementen, Lichtmodulatoren, Elementen zur Bildbearbeitung und/oder Signalverarbeitung oder allgemein im Bereich der nichtlinearen Optik.

Die Erfindung wird durch die nachfolgenden Beispiele näher erläutert.

### Beispiele

Für die ferroelektrischen Flüssigkristallmischungen werden die Werte für die spontane Polarisation Pₛ[nC/cm²] nach der Methode von H. Diamant et al. (Rev. Sci. Instr., 28, 30, 1957) in Meßzellen mit 10 µm Elektrodenabstand ohne Orientierungsschicht bei einer Temperatur von 25 °C gemessen.

Die kritische Pulsfläche (CPA) ist wie folgt definiert: Ein elektrischer Rechteckpuls geeigneter Spannung (Polarität) und Dauer kann bei gegebener Zellendicke den einen stabilen Schaltzustand in den anderen auf Dauer überführen, wenn Spannung und/oder Pulsdauer genügend groß sind. Hier ergibt sich die CPA aus dem Betrag des Produktes von 50 µsec Pulsdauer, der minimal notwendigen Spannung zum vollständigen dauerhaften Umschalten und dem Kehrwert der Zellendichte (T = 25 °C).

Der Schaltwinkel 2Θ_{eff} wird aus der Winkeldifferenz der beiden bisherigen Schaltzustände nach Kurzschließen der Elektroden bei 25 °C ermittelt. Für die Messungen von CPA und 2Θ_{eff} werden ca. 1,8 µm dicke Glastestzellen mit ITO-Elektroden und geriebener Polyvinylalkohol (PVA)-Orientierungsschicht verwendet. Die gefüllten Zellen werden bei 25°C 1 min lang mit Rechteckpulsen (IOH₂) bei einem elektrischen Feld von 15 V/µm geschaltet, bevor die eigentlichen Messungen stattfinden.

Die Phasenumwandlungstemperaturen werden beim Aufheizen und beim Abkühlen mit Hilfe eines Polarisationsmikroskops anhand der Texturänderungen bestimmt. Die Bestimmung des Schmelzpunkts wird hingegen mit einem DSC-Gerät durchgeführt. Die Angabe der Phasenumwandlungstemperaturen zwischen den Phasen
- Isotro: p (I)
- Nematisch: (N bzw. N*)
- Smektisch-A: (S_{A})
- Smektisch-C: (S_{c} bzw. S*_{c})
- Kristallin: (X)
erfolgt in °C, und die Werte stehen zwischen den Phasenbezeichnungen in der Phasenfolge. Es sind zunächst die Phasenübergänge beim Aufheizen in die isotrope Phase angegeben und dann die Phasenübergänge beim Abkühlen, wenn sie von denen des Aufheizens signifikant abweichen.

### Beispiel 1

### 2-(3-Methoxyphenyl)-5-(4-octyloxyphenyl)pyrimidin:

100 g (0,58 Mol) kommerziell erhältliches 3-Bromphenol in 600 ml Dimethylformamid werden bei Raumtemperatur portionsweise mit 25,43 g (0,64 Mol) einer 60 gew.-%igen Natriumhydriddispersion in Mineralöl versetzt und eine halbe Stunde nachgerührt, bevor 66,5 ml (0,58 Mol) kommerziell erhältliches Benzylchlorid zugetropft werden. Nach 1 h Rühren bei Raumtemperatur wird 1 l Wasser zugegeben, der dabei ausgefallene Feststoff abfiltriert, mit Wasser gewaschen und aus 600 ml Isopropanol umkristallisiert. Es werden 108 g 3-Benzyloxybrombenzol erhalten. 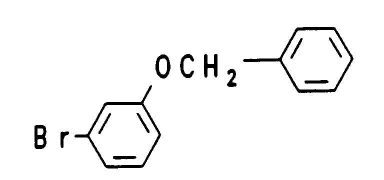 Aus 62 g (0,236 Mol) 3-Benzyloxybrombenzol, 6,64 g (0,277 Mol) Magnesium und 0,3 g Jod wird in 800 ml Tetrahydrofuran innerhalb von 2 h bei 60°C die Lösung der entsprechenden Grignardverbindung hergestallt, die anschließend zu einer auf 0°C gekühlten Lösung von 28,77 g (0,277 Mol) Trimethylborat in 300 ml Tetrahydrofuran getropft wird. Nach 1 h Rühren bei 0°C werden 74 ml 37 %ige Salzsäure in 300 ml Wasser zugegeben und anschließend das Reaktionsgemisch zwischen Wasser und Ether verteilt. Die organische Phase wird zweimal mit Wasser gewaschen, über Natriumsulfat getrocknet, eingeengt und aus Hexan umkristallisiert. Es werden 33,65 g
3-Benzyloxybenzolboronsäure erhalten.

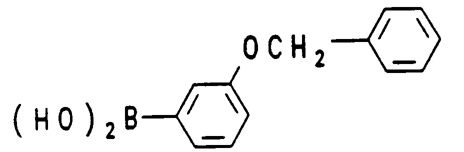

15,00 g (63,10 mmol) 2,5-Dibrompyrimidin (zur Herstellung siehe: D.W. Arantz und D.J. Brown in Journal of the Chemical Society C, 1971, S. 1889), 14,40 g (63,10 mmol) 3-Benzyloxybenzolboronsäure, 13,40 g (126,2 mmol) Natriumcarbonat und 0,73 g (0,63 mmol) Tetrakis(triphenylphosphin)palladium(0) werden in 150 ml Toluol, 75 ml Ethanol und 50 ml Wasser 4 h auf 80°C erhitzt. Anschließend wird zwischen Ether und Wasser verteilt, die organische Phase zweimal mit Natriumchloridlösung gewaschen, über Natriumsulfat getrocknet, eingeengt und chromatographisch (Kieselgel/Dichlormethan) gereinigt. Es werden 15,14 g 5-Brom-2-(3-benzyloxyphenyl)pyrimidin erhalten.

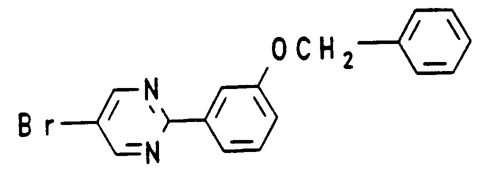

8,00 g (23,40 mmol) 5-Brom-2-(3-benzyloxyphenyl)pyrimidin, 7,03 g (28,10 mmol) 4-Octyloxy-benzolboronsäure (Herstellung analog 3-Benzyloxybenzolboronsäure), 7,00 g (65,52 mmol) Natriumcarbonat und 0,27 g (0,23 mmol) Tetrakis(triphenylphosphin)palladium(0) werden in 200 ml Toluol, 100 ml Ethanol und 50 ml Wasser 2 h auf 80°C erhitzt. Anschließend wird zwischen Wasser und Ether verteilt, die organische Phase zweimal mit Natriumchloridlösung gewaschen, über Natriumsulfat getrocknet, eingeengt und chromatographisch (Kieselgel/Dichlormethan: Ethylacetat = 98:2) gereinigt. Es werden 10,66 g 2-(3-Benzyloxyphenyl)-5-(4-octyloxyphenyl)pyrmidin erhalten.

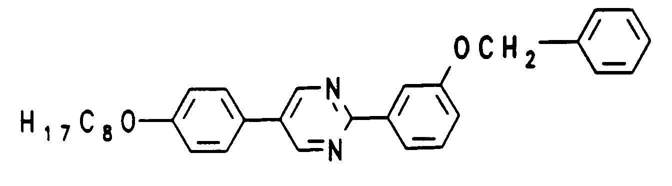

10,66 g (22,84 mmol) 2-(3-Benzyloxyphenyl)-5-(4-octyloxyphenyl)pyrimidin werden zusammen mit 2,00 g Pd (10 %)/C in 100 ml Tetrahydrofuran bis zum Verbrauch des berechneten Wasserstoffvolumens hydriert, anschließend wird vom Katalysator abfiltriert, eingeengt und getrocknet. Es werden 8,43 g 2-(3-Hydroxyphenyl)-5-(4-octyloxyphenyl)pyrimidin erhalten.

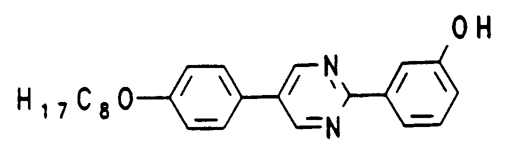

1,50 g (5,76 mmol) Triphenylphosphin und 0,90 ml (5,76 mmol) Azodicarbonsäurediethylester in 40 ml Tetrahydrofuran werden bei 0°C 30 min gerührt. Anschließend werden 1,50 g (3,84 mmol) 2-(3-Hydroxyphenyl)-5-(4-octyloxyphenyl)pyrimidin und 0,23 ml (5,76 mmol) Methanol zugegeben und die resultierende Lösung über Nacht bei Raumtemperatur gerührt. Nach Einengen zur Trockne, chromatographischer Reinigung (Kieselgel/Dichlormethan: Ethylacetat = 95:5) und Umkristallisation aus Acetonitril werden 1,20 g 2-(3-Methoxyphenyl)-5-(4-octyloxyphenyl)pyrimdin erhalten.

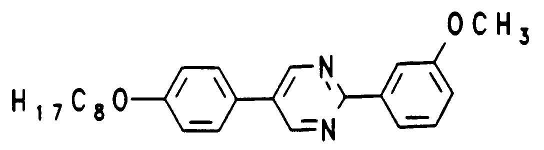

Die Verbindung zeigt die Phasenfolge:
X 70 S_{A} 98 I 98 S_{A} 37 X

Die Substanz wird im folgenden mit S₁ bezeichnet.

### Beispiel 2:

### 2-(3-Ethoxyphenyl)-5-(4-octyloxyphenyl)pyrimidin:

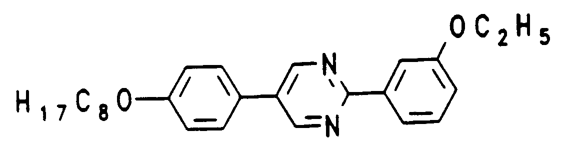

Die Synthese erfolgt analog Beispiel 1.

Die Verbindung zeigt die Phasenfolge:
X 83 I 71 S_{C} 60 X

Die Substanz wird im folgenden mit S₂ bezeichnet.

### Beispiel 3:

### 5-(4-octyloxyphenyl)-2-(3-propyloxyphenyl)pyrimidin:

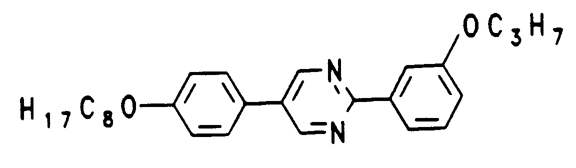

Die Synthese erfolgt analog Beispiel 1.

Die Verbindung zeigt die Phasenfolge:
X 84 I 76 S_{A} 61 S_{C} 50 X

Die Substanz wird im folgenden mit S₃ bezeichnet.

### Beispiel 4:

### 5-(4-Hexyloxyphenyl)-2-(3-propyloxyphenyl)pyrimidin:

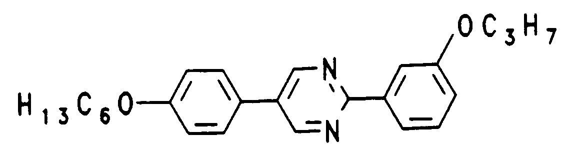

Die Synthese erfolgt analog Beispiel 1.

Die Verbindung zeigt die Phasefolge:
X 83 I 71,5 S_{A} 49 X

### Beispiel 5:

### 5-(4-Butyloxyphenyl)-2-(3-propyloxyphenyl)pyrimidin:

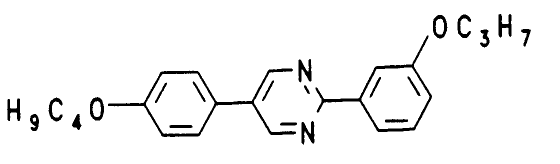

Die Synthese erfolgt analog Beispiel 1.

Die Verbindung zeigt die Phasenfolge:
X 95 I 95 S_{X} 57 X

### Beispiel 6:

### 2-(3-Hexyloxyphenyl)-5-(4-hexyloxyphenyl)pyrimidin:

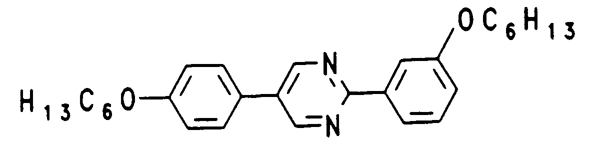

Die Synthese erfolgt analog Beispiel 1.

Die Verbindung zeigt die Phasenfolge:
X 87 S_{X} 88 I 75 S_{X} 59 X

### Beispiel 7:

### 5-(4-Butyloxyphenyl)-2-(3-hexyloxyphenyl)pyrimdin

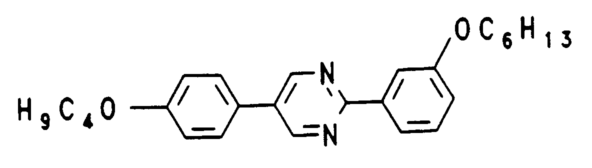

Die Synthese erfolgt analog Beispiel 1.

Die Verbindung zeigt die Phasenfolge:
X 86 I 75 S_{X} 65 X

### Beispiel 8:

### 5-(4-Decyloxyphenyl)-2-(3-propyloxyphenyl)pyrimidin:

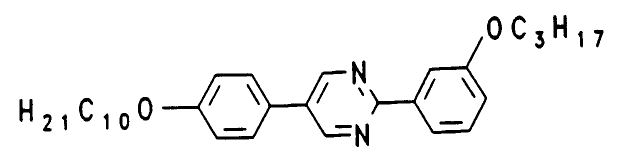

Die Synthese erfolgt analog Beispiel 1.

Die Verbindung zeigt die Phasenfolge:
X 78,4 I 76 S_{A} 60 S_{C} 41,5 X

### Beispiel 9:

### 2-(3-Cyclopropylmethoxyphenyl)-5-(4-octyloxyphenyl)pyrmidin

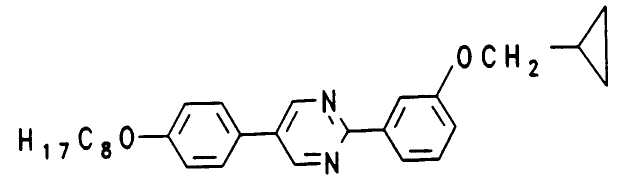

Die Synthese erfolgt analog Beispiel 1.

Die Verbindung zeigt die Phasenfolge:
X 98,1 I 58,6 X

### Beispiel 10:

### 2,5-Bis(3-propyloxyphenyl)pyrimidin:

Analog Beispiel 1 wird aus 3-Bromphenol und 1 -Brompropan 3-Propyloxybrombenzol hergestellt.

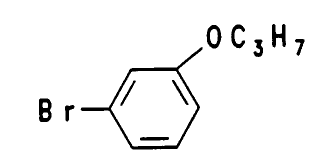

Analog Beispiel 1 wird aus 3-Propyloxybrombenzol 3-Propyloxybenzolboronsäure erhalten.

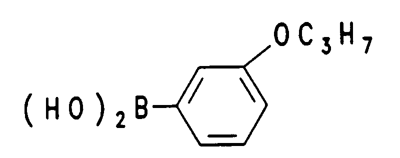

2,0 g (8,4 mmol) 2,5-Dibrompyrimdin, 4,54 g (25,2 mmol) 3-Propyloxybenzolboronsäure, 5,34 g (50,4 mmol) Natriumcarbonat und 0,10 g (0,9 mmol) Tetrakis(triphenylphosphin)palladium (0) werden in 50 ml Toluol, 25 ml Ethanol und 25 ml Wasser 6 h auf 80°C erhitzt. Anschließend wird zwischen Wasser und Ether verteilt, die organische Phase zweimal mit Natriumchloridlösung gewaschen, über Natriumsulfat getrocknet, eingeengt und chromatographisch (Kieselgel/Dichlormethan: Ethylacetat = 98:2) sowie durch Umkristallisation aus n-Hexan gereinigt. Es werden 1,31 g 2,5-Bis(3-propyloxyphenyl)pyrimidin erhalten.

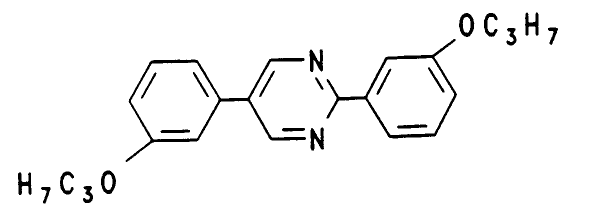

Die Verbindung zeigt die Phasenfolge:
X 81 I 31,6 X

### Beispiel 11:

### 2-(3-Methyloxyphenyl)-5-octyloxypyrimidin:

Zu 7,26 g (21,3 mmol) 2-(3-Benzyloxyphenyl)-5-brompyrimidin und 0,035 g (1,08 mmol) Schwefel in 60 ml Ethylenglykol werden bei 180°C über einen Zeitraum von 2 h eine Lösung von 4,76 g (85,3 mmol) Kaliumhydroxid in 20 ml Ethylenglykol getropft. Nach weiteren 3 h bei 180°C wird auf Wasser gegossen, mit 37 %iger Salzsäure angesäuert und der ausgefallene Feststoff durch Filtration isoliert. Nach chromatographischer Reinigung (Kieselgel/Hexan: Ethylacetat = 3:2) werden 4,77 g 2-(3-Benzyloxyphenyl)-5-hydroxypyrimidin erhalten.

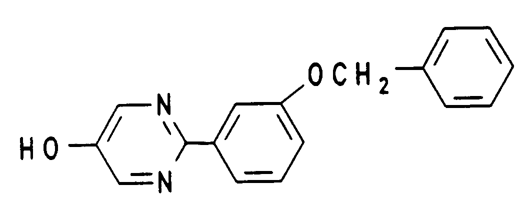

5,52g (21,05 mmol) Triphenylphosphin und 3,67g (21,05 mmol) Azodicarbonsäurediethylester werden in 100 ml Tetrahydrofuran 30 min bei 0°C gerührt. Anschließend werden 3,90 g (14,03 mmol) 2-(3-Benzyloxyphenyl)-5-hydroxypyrimidin und 2,74 g (21,05 mmol) 1-Octanol zugegeben und die resultierende Lösung 18 h bei Raumtemperatur gerührt. Nach dem Einengen zur Trockne und chromatographischer Reinigung (Kieselgel/Dichlormethan) werden 4,25 g 2-(3-Benzyloxyphenyl)-5-octyloxypyrimidin erhalten.

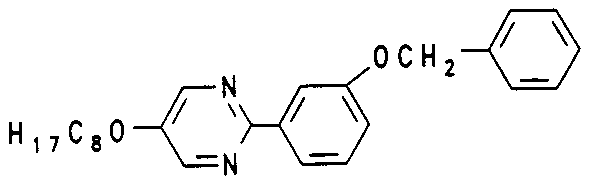

Analog Beispiel 1 wird durch Abhydrierung der Benzylschutzgruppe 2-(3-Hydroxyphenyl)-5-octyloxypyrimidin erhalten.

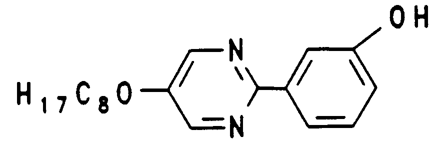

Analog Beispiel 1 wird aus 2-(3-Hydroxyphenyl)-5-octyloxypyrimidin durch Umsetzung mit Methanol 2-(3-Methyloxyphenyl)-5-octyloxypyrimidin erhalten.

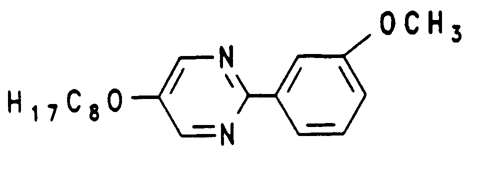

Die Verbindung zeigt die Phasenfolge:
X 43 I

### Beispiel 12

### 2-(3-Ethyloxyphenyl)-5-octyloxypyrimidin:

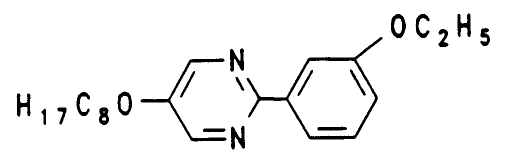

Die Synthese erfolgt analog Beispiel 11.

Die Verbindung zeigt die Phasenfolge:
X 50 I

### Beispiel 13:

### 5-Octyloxy-2-(3-propyloxyphenyl)pyrimidin:

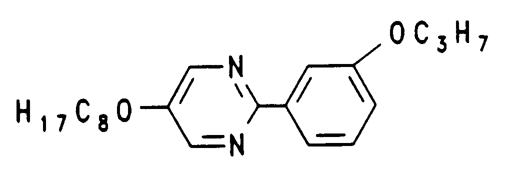

Die Synthese erfolgt analog Beispiel 11.

Die Verbindung zeigt die Phasenfolge:
X 56 I

### Beispiel 14

### 5-(3-Methoxyphenyl)-2-(4-octyloxyphenyl)pyrimidin:

Analog Beispiel 1 wird aus 2,5-Dibrompyrimidin und 4-Octyloxybenzolboronsäure 5-Brom-2-(4-octyloxyphenyl)pyrimidin hergestellt.

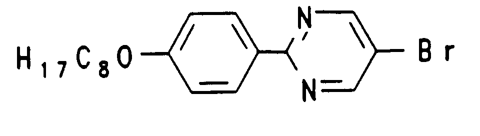

Analog Beispiel 1 wird aus 5-Brom-2-(4-octyloxyphenyl)pyrimidin und 3-Benzyloxybenzolboronsäure 5-(3-Benzyloxyphenyl)-2-(4-octyloxyphenyl)pyrimidin erhalten.

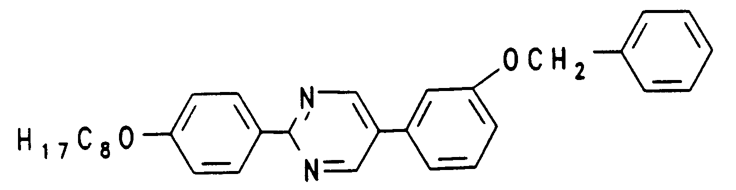

Die Abhydrierung der Benzylschutzgruppe von 5-(3-Benzyloxyphenyl)-2-(4-octyloxyphenyl)pyrimidin analog Beispiel 1 führt zu 5-(3-Hydroxyphenyl)-2-(4-octyloxyphenyl)pyrimidin.

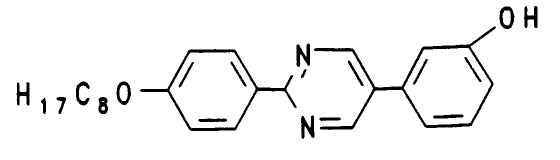

Analog Beispiel 1 wird aus 5-(3-Hydroxyphenyl)-2-(4-octyloxyphenyl)pyrimidin durch Umsetzung mit Methanol 5-(3-Methoxyphenyl)-2-(4-octyloxyphenyl)pyrimidin erhalten.

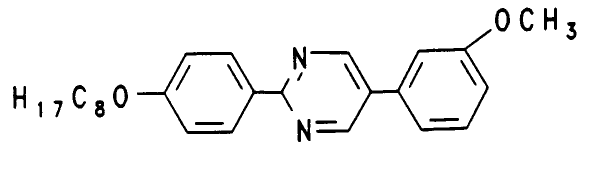

Die Verbindung zeigt die Phasenfolge:
X 98 I

### Beispiel 15:

### 5-(3-Ethoxyphenyl)-2-(4-octyloxyphenyl)pyrimidin:

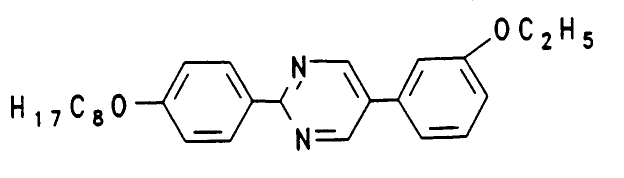

Die Synthese erfolgt analog Beispiel 14.

Die Verbindung zeigt die Phasenfolge:
X 108 I

### Beispiel 16:

### 2-(4-Octyloxyphenyl)-5-(3-propyloxyphenyl)pyrimidin:

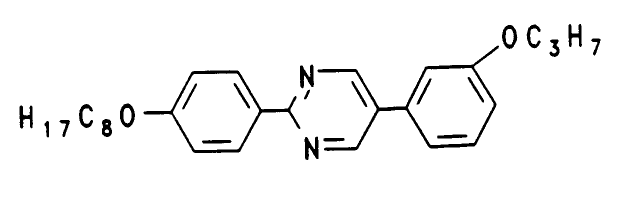

Die Synthese erfolgt analog Beispiel 14.

Die Verbindung zeigt die Phasenfolge:
X 107 I

### Beispiel 17:

### 5-[4-((2S,3S)-3-Butyloxiran-2-ylmethoxy)phenyl]-2-(3-propyloxyphenyl)pyrimidin:

Analog Beispiel 1 wird aus 2,5-Dibrompyrimidin und 3-Propyloxybenzolboronsäure 5-Brom-2-(3-propyloxyphenyl)pyrimidin hergestellt.

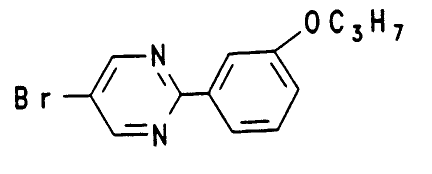

Aus 5-Brom-2-(3-propyloxyphenyl)pyrimidin und 4-Benzyloxybenzolboronsäure (Herstellung analog 3-Benzyloxybenzolboronsäure) wird analog Beispiel 1 5-(4-Benzyloxyphenyl)-2-(3-propyloxyphenyl)pyrimidin erhalten.

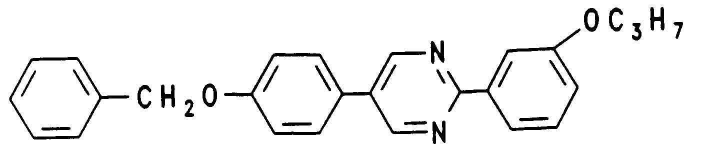

Durch Abhydrierung der Benzylschutzgruppe analog Beispiel 1 wird aus obiger Verbindung 5-(4-Hydroxyphenyl)-2-(3-propyloxyphenyl)pyrimidin hergestellt.

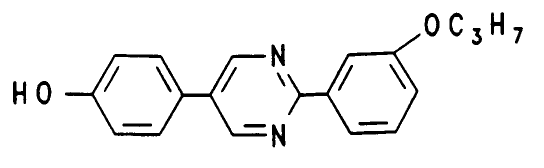

Analog Beispiel 1 wird aus 5-(4-Hydroxyphenyl)-2-(3-propyloxyphenyl)pyrimidin und (2S,3S)-3-Butyloxiran-2-ylmethanol (Herstellung beschrieben in EP-0 046 033 und Journal of the American Chemical Society Band 109, 1987, seite 5765) 5-[4-((2S,3S)-3-Butyloxiran-2-ylmethoxy)phenyl]-2-(3-propyloxyphenyl)pyrimidin synthetisiert. 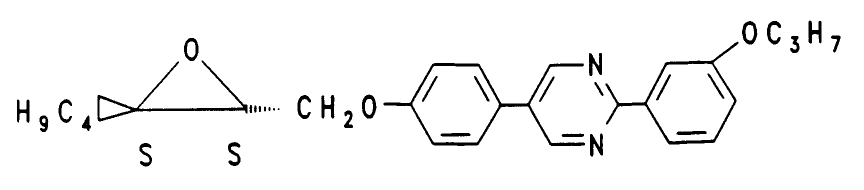
[α]$\frac{\text{20}}{\text{D}}$ (CHCl3) = -17,25

Die Verbindung zeigt die Phasenfolge:
X 89,7 I 84 S_{A} 53 S_{Z} 49,8 X

### Beispiel 18:

### 5-[3-((2S,3S)-3-Butyloxiran-2-ylmethoxy)phenyl]-2-(4-octyloxyphenyl)pyrimidin:

Die Synthese erfolgt analog Beispiel 1 aus 5-(3-Hydroxyphenyl)-2-(4-octyloxyphenyl)pyrimidin und (2S,3S)-3-Butyloxiran-2-ylmethanol. 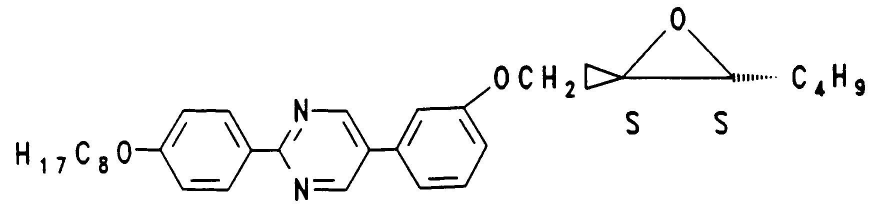
[α]$\frac{\text{20}}{\text{D}}$ (CHCl₃) = -14,27

Die Verbindung zeigt die Phasenfolge:
X 91 I

### Beispiel 19:

### 2-[3-((2S,3S)-3-Butyloxiran-2-ylmethoxy)phenyl]-5-octyloxypyrimidin:

Die Synthese erfolgt analog Beispiel 11 aus 2-(3-Hydroxyphenyl)-5-octyloxypyrimidin und (2S,3S)-3-Butyloxiran-2-ylmethanol. 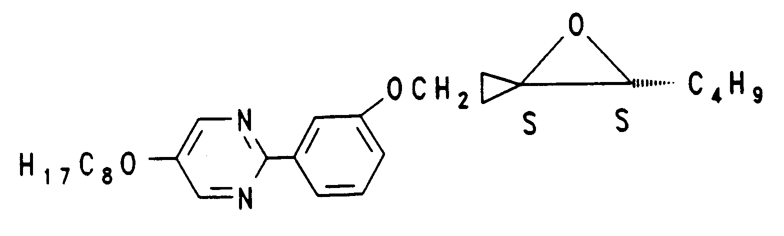
[α]$\frac{\text{20}}{\text{D}}$ (CHCl₃) = -14,74

Die Verbindung zeigt die Phasenfolge:
X 48 I

### Beispiel 20

### 5-[4-((4S)-2,2-Dimethyl-1,3-dioxolan-4-ylmethoxy)phenyl]-2-(3-propyloxyphenyl)pyrimidin:

Die Synthese erfolgt analog Beispiel 17 aus 5-(4-Hydroxyphenyl)-2-(3-propyloxyphenyl)pyrimidin und (4S)-2,2-Dimethyl-1,3-dioxolan-4-ylmethanol (kommerziell erhältlich). 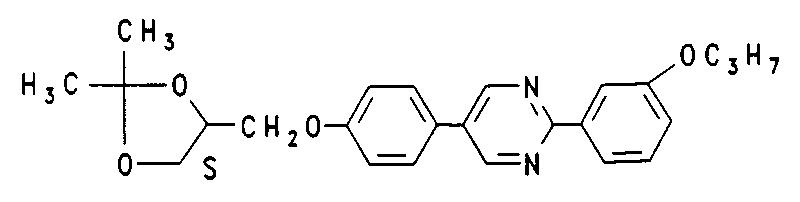
[α]$\frac{\text{20}}{\text{D}}$ (CHCl₃) = + 5,0

Die Verbindung zeigt die Phasenfolge:
X 107,1 I 85,0 X

### Beispiel 21

### (2R,3R)-3-Propyloxiran-2-carbonsäure-4-[2-(3-propyloxyphenyl)pyrimidin-5-yl]phenylester:

1,5 g (4,90 mmol) 5-(4-Hydroxyphenyl)-2-(3-propyloxyphenyl)pyrimidin, 1,12 g (7,4 mmol) (2R,3R)-3-Propyloxiran-2-carbonsäure-Natriumsalz (hergestellt wie in DE 43 04 756 beschrieben), 2,80 g (7,4 mmol) Hydroxybenzotriazol-tetramethyl-uroniumhexafluorophosphat und 0,81 ml (7,4 mmol) N-Methylmorpholin werden in 40 ml Acetonitril bei Raumtemperatur unter Lichtausschluß 17 h gerührt. Zur Aufarbeitung wird zwischen Dichlormethan und wäßriger Natriumhydrogencarbonatlösung verteilt, die organische Phase mit wäßriger Natriumchloridlösung gewaschen, über Magnesiumsulfat getrocknet, filtriert und eingeengt. Nach Reinigung durch Chromatographie (Kieselgel/Dichlormethan: Ethylacetat = 20:1) und Umkristallisation aus Acetonitril werden 0,9 g Produkt erhalten. 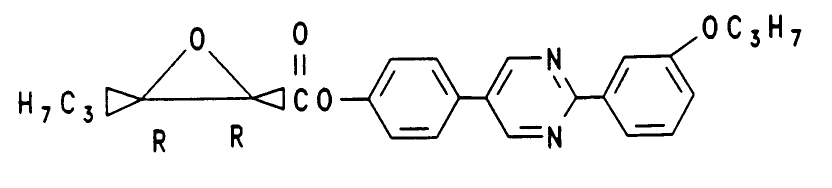
[α]$\frac{\text{20}}{\text{D}}$ (CHCl₃) = -13,0

Die Verbindung zeigt die Phasenfolge:
X 116 I 82 X

### Beispiel 22

### (2R,3R)-3-Propyloxiran-2-carbonsäure-3-[5-(4-octyloxyphenyl)pyrimidin-2-yl]phenylester: Die Synthese erfolgt analog Beispiel 21.

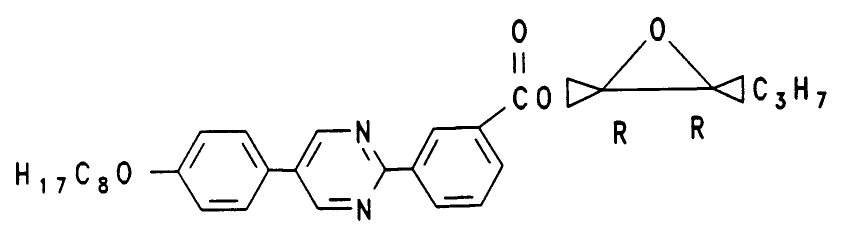

[α]$\frac{\text{20}}{\text{D}}$ (CHCl₃) = + 0,33

Die Verbindung zeigt die Phasenfolge:
X 111 I

### Beispiel 23:

### (4S)-2,2-Dimethyl-1,3-dioxolan-4-carbonsäure-4-[2-(3-propyloxyphenyl)pyrimidin-5-yl]phenylester:

Die Synthese erfolgt analog Beispiel 21 unter Verwendung von (4S)-2,2-Dimethyl-1,3-dioxolan-4-carbonsäure-Natriumsalz, dessen Herstellung in DE 43 04 756 beschrieben ist. 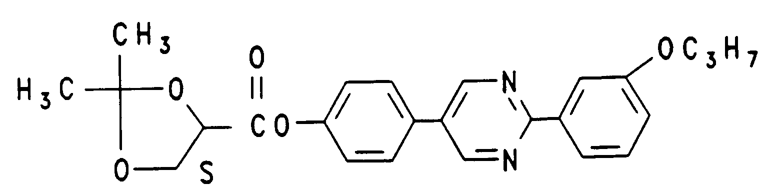
[α]$\frac{\text{20}}{\text{D}}$ (CHCl₃) = +7,1

Die Verbindung zeigt die Phasenfolge:
X₁ 129 X₂ 136 I 112 X

### Beispiel 24:

### (4S)-2,2-Dimethyl-1,3-dioxolan-4-carbonsäure-3-[5-(4-octyloxyphenyl)pyrimidin-2-yl]phenylester

Die Synthese erfolgt analog Beispiel 23. 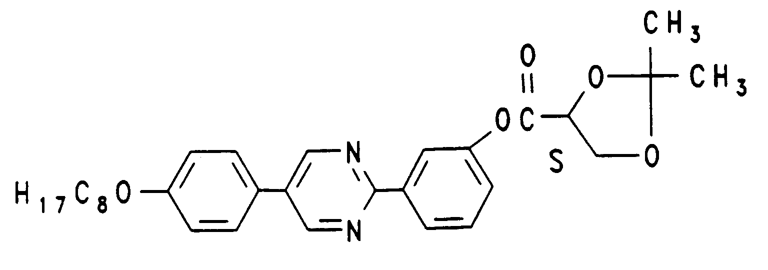
[α]$\frac{\text{20}}{\text{D}}$ (CHCl₃) = +12,81

Die Verbindung zeigt die Phasenfolge:
X 135 I

### Beispiel 25:

### 2-(3-Propyloxyphenyl)-5-(4-octyloxyphenyl)pyridin:

Die Synthese erfolgt analog Beispiel 1 unter Verwendung des kommerziell erhältlichen 2,5-Dibrompyridins anstelle von 2,5-Dibrompyrimidin.

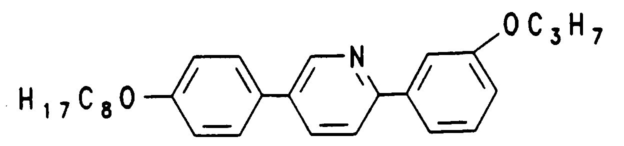

### Beispiel 26:

### 2-(3-Propyoxyphenyl)-5-(4-octyloxyphenyl)pyrazin:

Die Synthese erfolgt analog Beispiel 1 unter Verwendung von 2,5-Dibrompyrazin (zur Herstellung siehe: R.C.Ellingson und R.L.Henry in Journal of the American Chemical Society, Band 71 (1949), Seite 2798 bis 2800) anstelle von 2,5-Dibrompyrimidin.

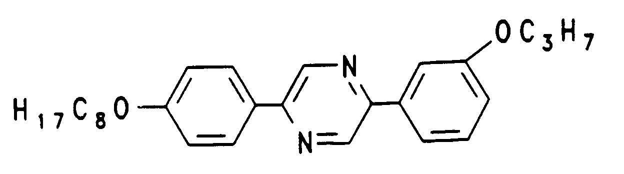

### Beispiel 27:

### 3-(4-Octyloxyphenyl)-6-(3-propyloxyphenyl)pyridazin:

Die Synthese erfolgt analog Beispiel 1 unter Verwendung des kommerziell erhältlichen 3,6-Dichlorpyridazins anstelle von 2,5-Dibrompyrimidin.

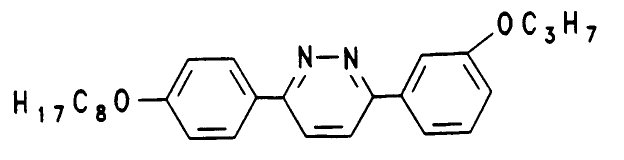

### Beispiel 28:

### 2-(4-Octyloxyphenyl)-5-(3-propyloxyphenyl)thiadiazol:

Die Synthese erfolgt analog der von K.Dimitrova, J.Hauschild, H.Zaschke und H.Schubert im Journal für praktische Chemie, Band 322 (1980), Seite 933 beschriebenen Methode.

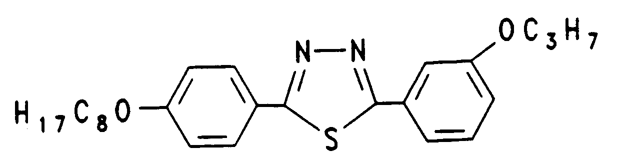

### Beispiel 29:

### 4-Octyloxy-3''-propyloxy[1,1',4',1'']terphenyl:

Die Synthese erfolgt analog Beispiel 1 unter Verwendung des kommerziell erhältlichen 1,4-Dibrombenzols anstelle von 2,5-Dibrompyrimidin.

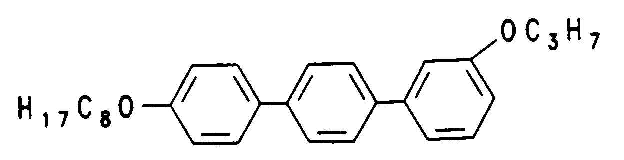

### Beispiel 30:

### 2',3'-Difluor-4-octyloxy-3''-propyloxy[1,1',4',1'']terphenyl:

Die Synthese erfolgt analog Beispiel 1 unter Verwendung des kommerziell erhältlichen 1,4-Dibrom-2,3-difluor-benzols anstelle von 2,5-Dibrompyrimidin.

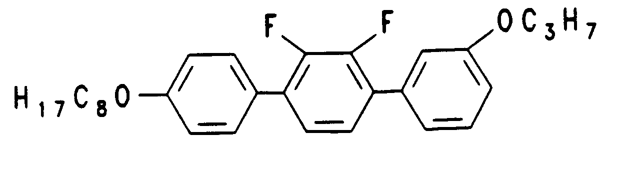

### Beispiel 31:

### 2-(2-Fluor-3-propyloxyphenyl)-5-(4-octyloxyphenyl)pyrimidin:

Die Synthese erfolgt analog Beispiel 1 unter Verwendung von 3-Brom-2-fluorphenol anstelle von 3-Bromphenol.

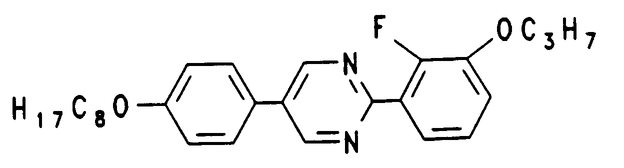

### Anwendungsbeispiele: 1/2/3

Die eingesetzte FLC-Ausgangsmischung Mₒ (enthaltend keine erfindungsgemäßen Substanzen) mit einer Phasenfolge X-4,5(-10) S_{C}* 63 S_{A} 69 N* 83 I besitzt folgende Zusammensetzung (in mol-%):

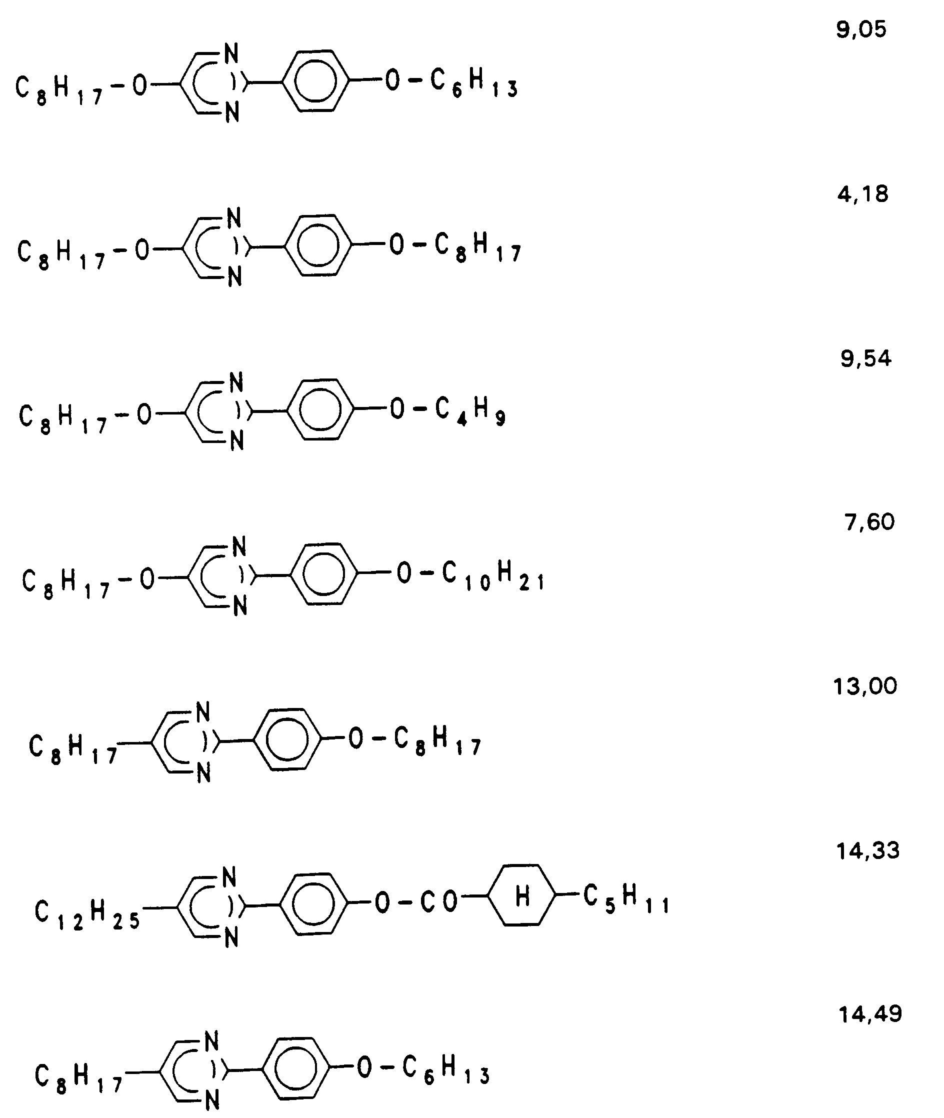

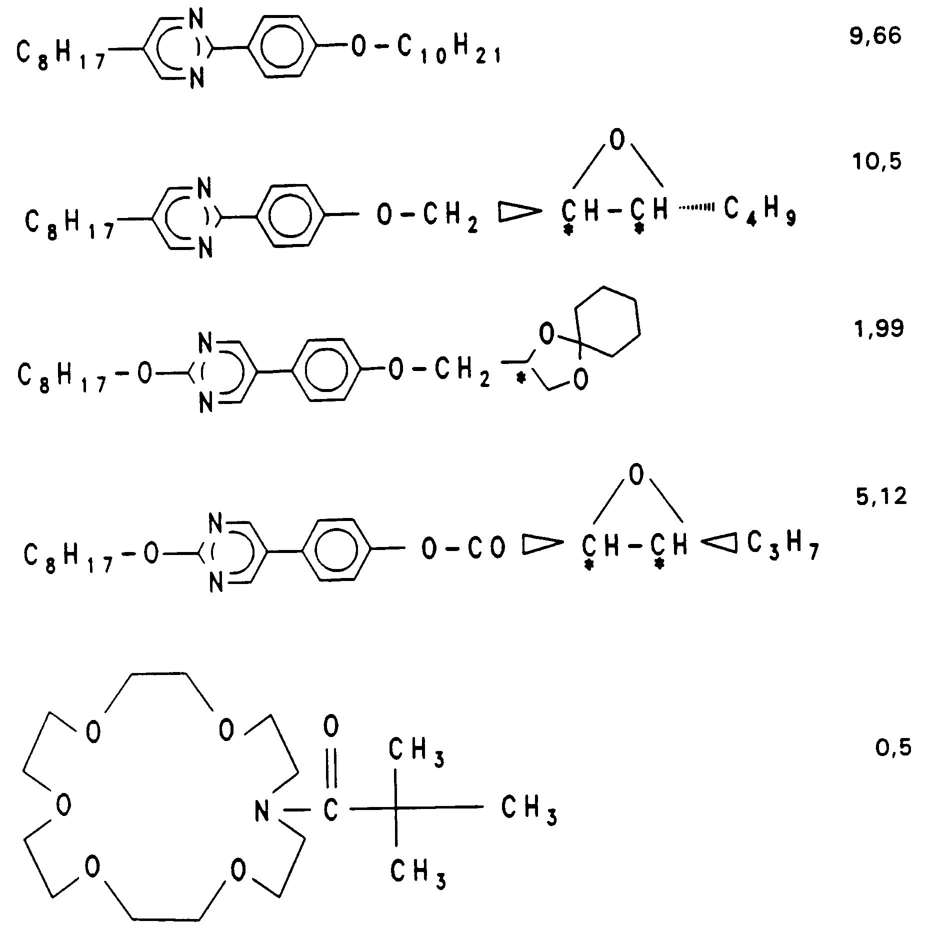

Die eingesetzten Mischungen M₁, M₂ und M₃ besitzen folgende

Zusammensetzung und zeigen die angegebenen Phasenfolgen:
- M₁:: 95 mol-% Mₒ + 5 mol-% S₁
S_{C}* 60 S_{A} 74 N* 84 I
- M₂:: 95 mol-% Mₒ + 5 mol-% S₂
S_{C}* 59 S_{A} 74 N* 84 I
- M₃:: 95 mol-% Mₒ + 5 mol-% S₃
X-9(-17) S_{C}* 59 S_{A} 74 N* 83 I

Man erkennt, daß durch Zugabe der Substanzen S₁, S₂ und S₃ der Phasenübergang N*-I tendentiell erhöht wird. Dies unterstreicht die gute Eignung der erfindungsgemäßen Substanzen als Komponenten von Flüssigkristallmischungen.

In der folgenden Tabelle sind die Meßwerte für Pₛ, 2Θ_{eff} und die CPA bei 25°C der Mischungen Mₒ, M₁, M₂ und M₃ dargestellt.

| Mischung | Pₛ nC ⁻¹ cm² | 2Θ_{eff} [°]⁻¹ | CPA Vs ⁻¹ m |
|---|---|---|---|
| Mₒ | 33 | 49 | 900 |
| M₁ | 29 | 42 | 730 |
| M₂ | 29 | 42 | 630 |
| M₃ | 30 | 43 | 710 |

Man erkennt, wie günstig die Substanzen auf Schaltwinkel und CPA wirken. Der Schaltwinkel wird auf Werte nur wenig unter 45° (physikalisch ideal) gesenkt, was als technisch hervorragend angesehen werden kann. Die Substanzen S₁ und S₃ reduzieren die CPA um etwa 20 %, die Substanz S₃ sogar um 30 %. Dies wirkt sich entweder positiv auf die nötige Treiberspannung eines Displays - bei konstanter Pulsbreite - oder vorteilhaft auf die Pulsbreite bei konstanter Treiberspannung aus.

Darüberhinaus zeigt sich, daß die erfindungsgemäßen Substanzen den Schmelzpunkt stark senken können.

### Anwendungsbeispiel 4

Der eingesetzten FLC-Ausgangsmischung Mₒ aus Beispiel 1, 2 und 3 werden 5 mol-% der erfindungsgemäßen Substanz aus Beispiel 9 zugegeben. Es wird folgende Phasenfolge bestimmt: X-9(-16) S_{C}* 57,5 S_{A} 72,5 N* 82 I. Bei 25°C wird eine CPA = 640 Vs/m bei einer Polarisation P_{S} = 40 nC/cm² und einem Winkel von 45° (idealer Wert) gemessen. Wieder zeigt sich der günstige Einfluß auf Schaltgeschwindigkeit und Winkel. Darüber hinaus wird der Schmelzpunkt der Mischung deutlich gesenkt.

### Anwendungsbeispiel Nr. 5

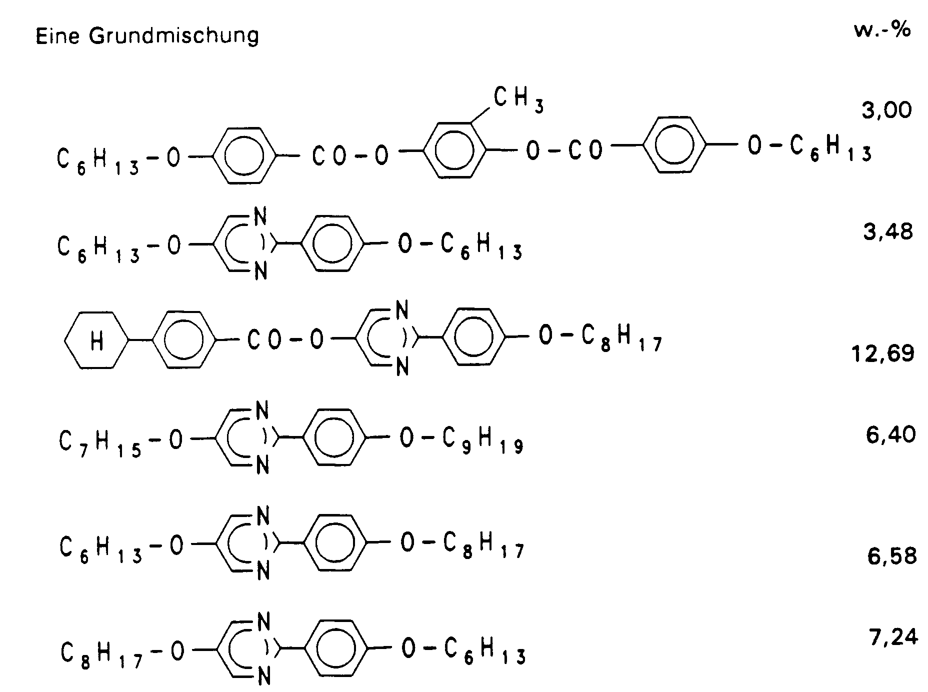

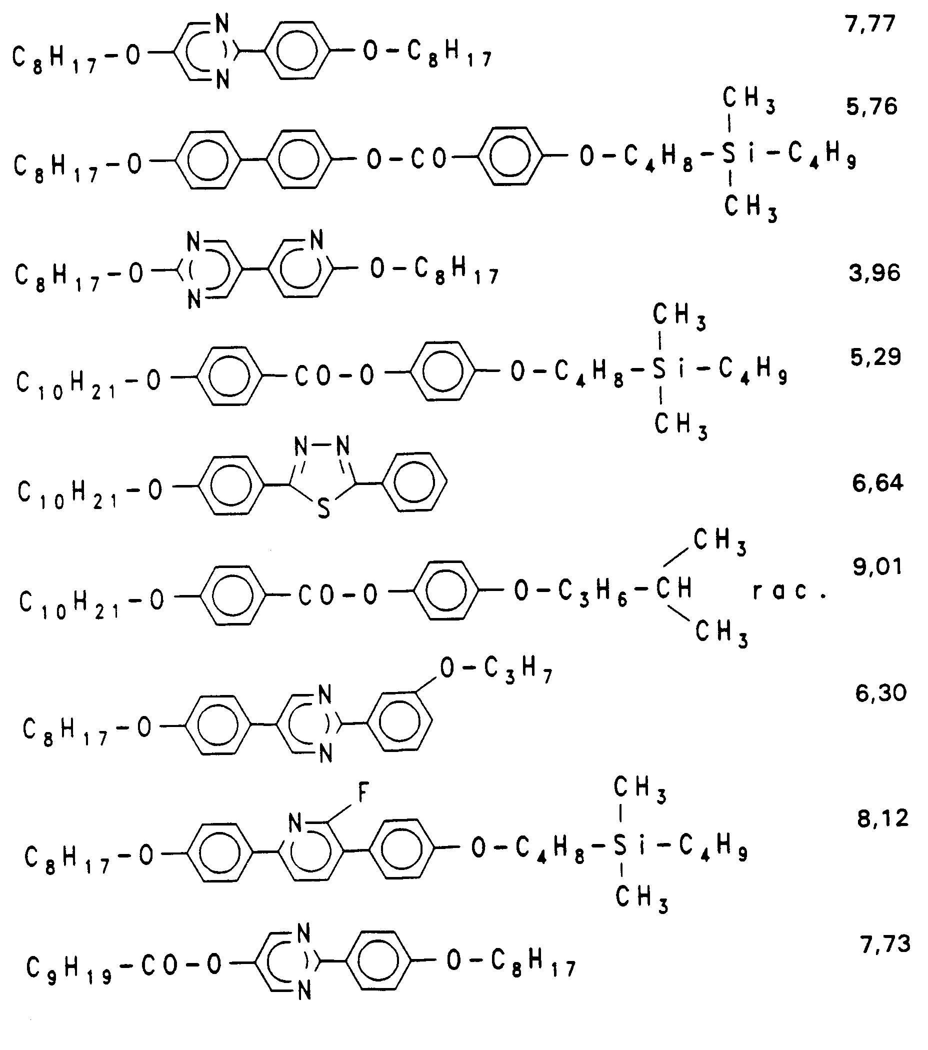

zeigt die Phasenfolge:
X -21(-40) S_{C} 79 S_{A} 91 N 102 I

Zugegeben werden 10 Gew.-% der Substanz aus Beispiel 9
- Phasenfolge:: X -35(-41) S_{C} 71 S_{A} 85 N 98 I

Es wird der günstige Einfluß auf den Schmelzpunkt deutlich.

### Anwendungsbeispiel Nr. 6

In die Grundmischung aus Anwendungsbeispiel Nr. 5 wird die Substanz aus Beispiel 17 zu 10 Gew.-% zugegeben. Phasenfolge: X -41 (< -45) S_{C} 66 S_{A} 96 N 98 I Auch hier wird der guten Einfluß auf den Schmelzpunkt wiederum klar, besonders wenn man den schon sehr niedrigen Schmelzpunkt der Ausgangsmischung berücksichtigt.

### Anwendungsbeispiel Nr. 7

In die Grundmischung aus Anwendungsbeispiel 5 wird die Substanz aus Beispiel 21 zugegeben (5 Gew.-%).
- Phasenfolge:: X 60 (< -45) S_{C}* 71 S_{A} 81 N* 95 I

Ps (20°C) = 31,1 nC/cm²
Ps (40°C) = 22,6 nC/cm²
Ps (60°C) = 13,0 nC/cm²

Es zeigt sich eine sehr hohe Polarisation schon bei kleiner Dotierstoffmenge.

## Patentansprüche

1. Verbindungen der Formel (I) 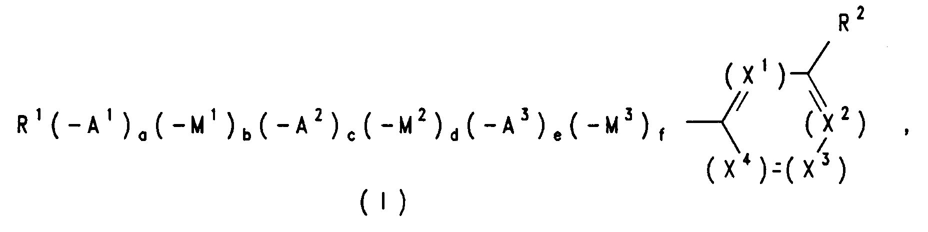 in der die Symbole und Indizes folgende Bedeutung haben:
R¹, R² sind unabhängig voneinander ein geradkettiger oder verzweigter Alkylrest mit 1 bis 22 C-Atomen (mit oder ohne asymmetrische C-Atome), wobei auch eine oder zwei CH₂-Gruppen durch -O-, -S-, -CO-, -CO-O-, -O-CO-, -CO-S-, -S-CO-, -O-CO-O-, -CH=CH-, -C≡C-, Cyclopropyldiyl oder -Si(CH₃)₂- ersetzt sein können und wobei auch ein oder mehrere H-Atome des Alkylrestes durch F, Cl, Br oder CN substituiert sein können, oder eine der nachfolgenden chiralen Gruppen: 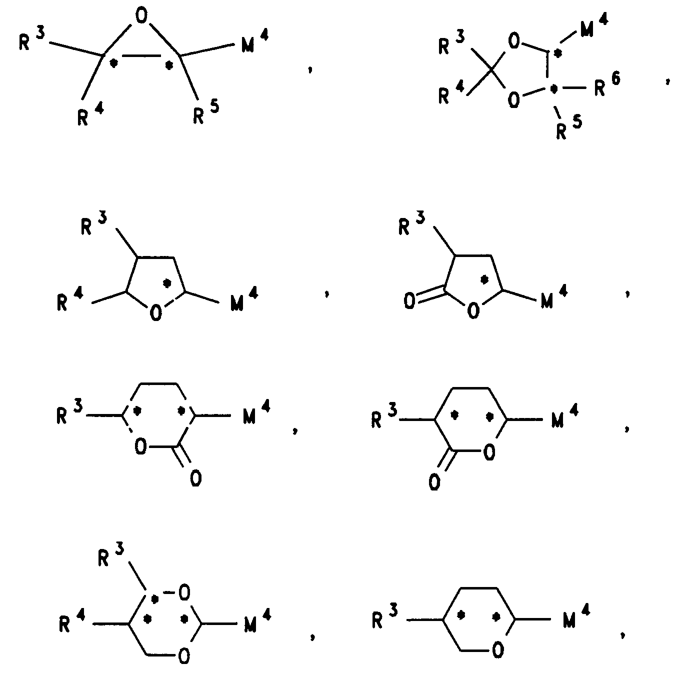 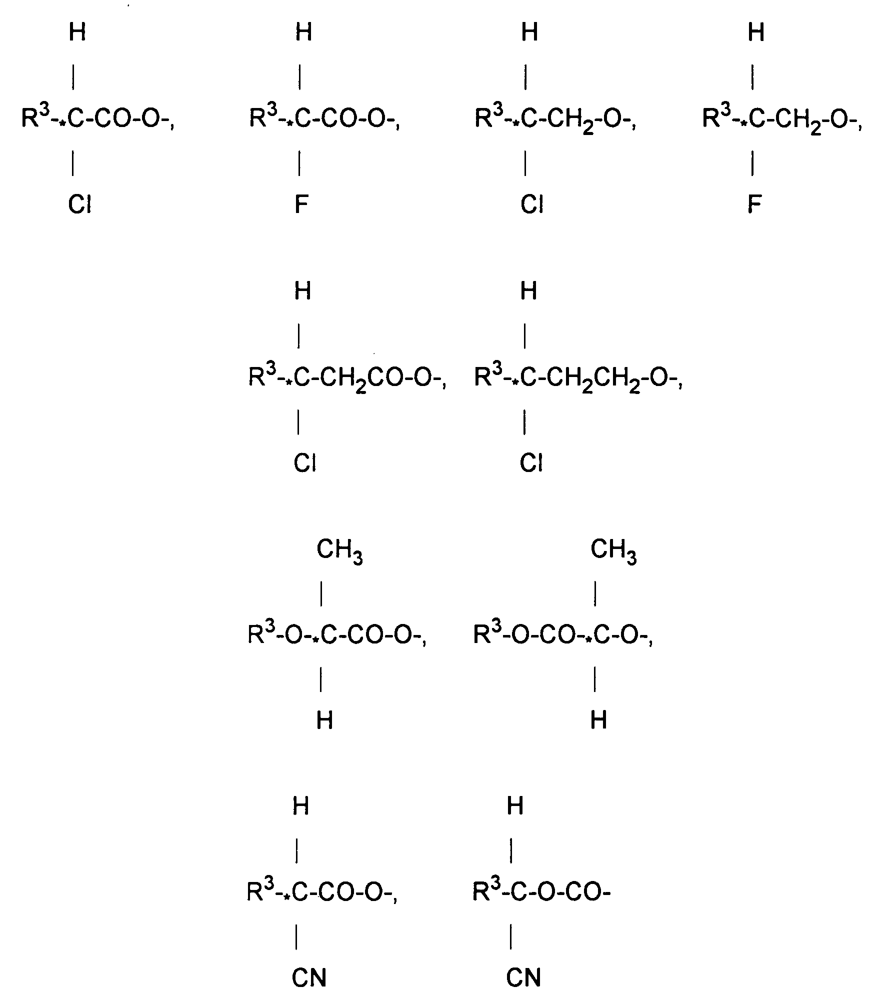
R³, R⁴, R⁵, R⁶ sind unabhängig voneinander H oder ein geradkettiger oder verzweigter Alkylrest mit 1 bis 22 C-Atomen, wobei auch eine oder zwei CH₂-Gruppen durch -O-, -S-, -CO-, -CO-O, -O-CO-, -CO-S-, -S-CO-, -O-CO-O-, -CH=CH-, -C≡C-, Cyclopropyldiyl oder -Si(CH₃)₂- ersetzt sein können, oder R³ und R⁴ können zusammen auch -(CH₂)₄- oder -(CH₂)₅- sein, wenn sie als Substituenten an ein Dioxolan-System gebunden sind;
A¹, A², A³ sind unabhängig voneinander 1,4-Phenylen, wobei ein oder zwei H-Atome durch F, Cl und/oder CN ersetzt sein können, Pyrazin-2,5-diyl, Pyridazin-3,6-diyl, Pyridin-2,5-diyl, Pyrimidin-2,5-diyl, bei denen ein oder zwei H-Atome durch F ersetzt sein können, trans-1,4-Cyclohexylen, bei dem ein oder zwei H-Atome durch -CN und/oder -CH₃ ersetzt sein können, (1,3,4)-Thiadiazol-2,5-diyl, 1,3-Dioxan-2,5-diyl, 1,3-Dithian-2,5-diyl, 1,3-Thiazol-2,4-diyl, 1,3-Thiazol-2,5-diyl, Thiophen-2,4-diyl, Thiophen-2,5-diyl, Piperazin-1,4-diyl, Piperazin-2,5-diyl, Naphthalin-2,6-diyl, Bicyclo[2.2.2]octan-1,4-diyl, 1,3-Dioxaborinan-2,5-diyl oder trans-Dekalin-2,6-diyl;
A¹ kann auch eine Gruppe der Formel 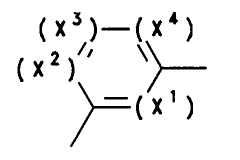 sein;
M¹, M², M³ sind unabhängig voneinander -O-, -S-, -CO-, -CO-O-, -O-CO-, -CO-S-, -S-CO-, -O-CO-O-, -CH₂-O-, -O-CH₂-, -CH₂-CH₂-, -CH=CH- oder -C≡CH-;
M⁴ ist -CH₂-O-, -O-CH₂-, -CO-O-, -O-CO- oder ein Einfachbindung;
X¹, X², X³, X⁴ sind CH, CF oder N, wobei die Zahl der N-Atome 0, 1 oder 2 beträgt;
a, b, c, d, e, f sind null oder eins, unter der Bedingung, daß die Summe a + c + e 1, 2 oder 3 ist;
* ist ein chirales Zentrum,
wobei Verbindungen mit
M³: -COO-, -O-CO-, -O-CH₂- oder -CH₂O-;
f :1; und 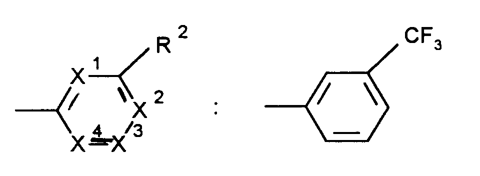 ausgenommen sind.

2. Verbindungen nach Anspruch 1, wobei die Symbole und Indices folgende Bedeutung haben:
R¹, R² sind unabhängig voneinander ein geradkettiger oder verzweigter Alkylrest mit 1 bis 15 C-Atomen (mit oder ohne asymmetrische C-Atome), wobei auch eine oder zwei CH₂-Gruppen durch -O-, -S-, -CO-, -CO-O-, -O-CO-, -OCO-O-, -CH=CH-, -C≡C-, Cyclopropyldiyl oder -Si(CH₃)₂- ersetzt sein können, oder eine der nachfolgenden chiralen Gruppen: 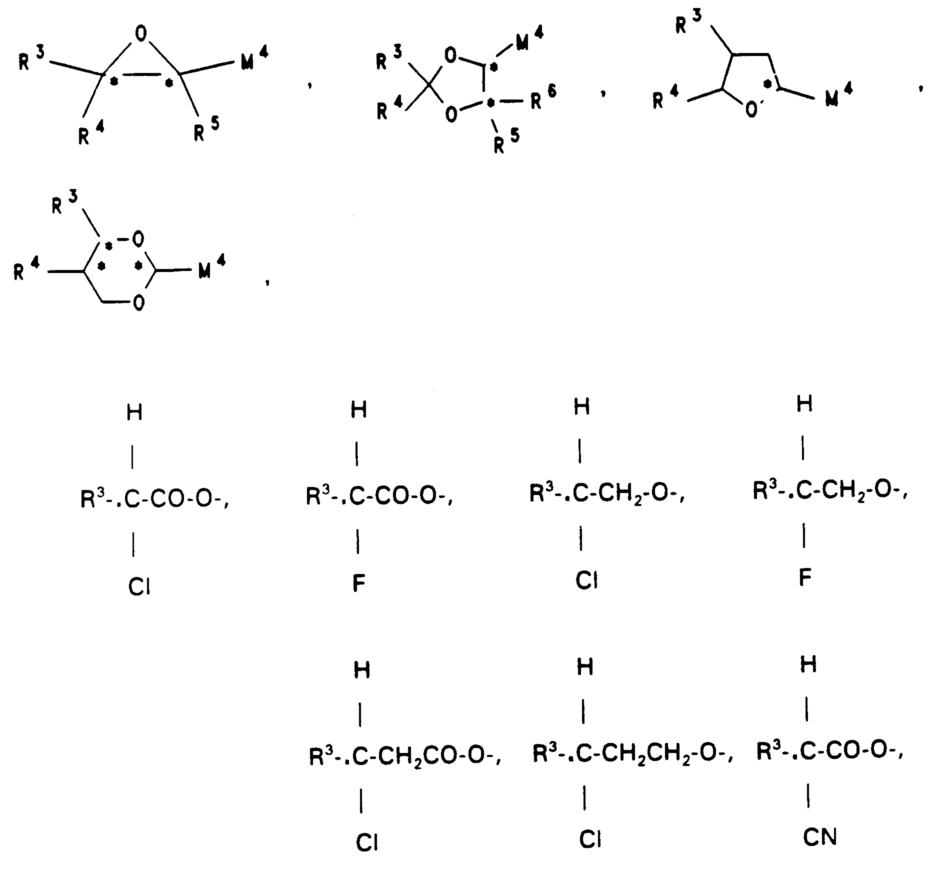 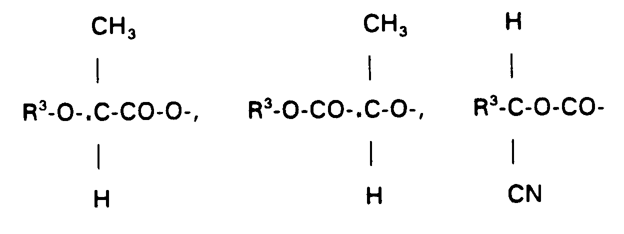
R³, R⁴, R⁵, R⁶ sind unabhängig voneinander H oder ein geradkettiger oder verzweigter Alkylrest mit 1 bis 15 C-Atomen, wobei auch eine oder zwei CH₂-Gruppen durch -O-, -S-, -CO-, -CO-O-, -O-CO-, -CO-S-, -S-CO-, -O-CO-O-, -CH=CH-, -C≡C, Cyclopropyldiyl oder -Si(CH₃)₂- ersetzt sein können, oder R³ und R⁴ können zusammen auch -(CH₂)₄- oder -(CH₂)₅-sein, wenn sie als Substituenten an ein Dioxolan-System gebunden sind;
A¹, A², A³ sind unabhängig voneinander 1,4-Phenylen, Pyrazin-2,5-diyl, Pyridazin-3,6-diyl, Pyridin-2,5-diyl, Pyrimidin-2,5-diyl, bei denen ein oder zwei H-Atome durch F ersetzt sein können, trans-1,4-Cyclohexylen, (1,3,4)-Thiadiazol-2,5-diyl, 1,3-Dioxan-2,5-diyl, Naphthalin-2,6-diyl oder Bicyclo[2.2.2]octan-1,4-diyl;
A¹ kann auch eine Gruppe der Formel 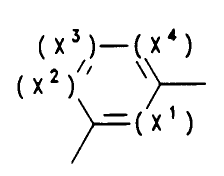 sein;
M¹, M², M³ sind unabhängig voneinander -O-, -CO-, -CO-O-, -O-CO-, -CH₂-O-, -O-CH₂-, -CH₂CH₂-, -CH=CH- oder -C≡C-;
M⁴ ist -CH₂-O-, -O-CH₂-, -CO-O-, -O-CO- oder eine Einfachbindung;
X¹, X², X³, X⁴ sind CH, CF oder N, wobei die Zahl der N-Atome 0, 1 oder 2 beträgt.

3. Verbindung nach Anspruch 1, wobei die Symbole und Indizes folgende Bedeutung haben:
R¹, R² sind unabhängig voneinander ein geradkettiger oder verzweigter Alkylrest mit 1 bis 10 C-Atomen (mit oder ohne asymmetrische C-Atome), wobei auch eine oder zwei CH₂-Gruppen durch -O-, -CO-, -CO-O-, -O-CO-, -CH=CH-, -C≡C-, Cyclopropyldiyl oder -Si(CH₃)₂-ersetzt sein können, oder eine der nachfolgenden chiralen Gruppen: 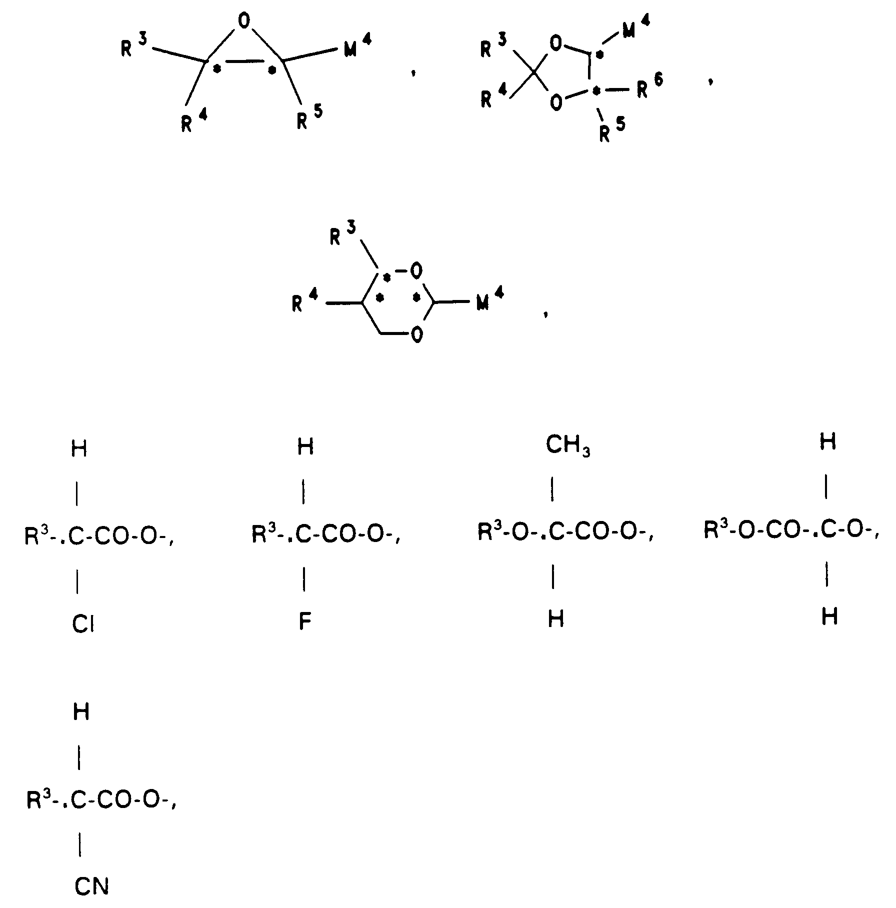
R³, R⁴, R⁵, R⁶ sind unabhängig voneinander H oder ein geradkettiger oder verzweigter Alkylrest mit 1 bis 10 C-Atomen, wobei auch eine oder zwei CH₂-Gruppen durch -O-, -S-, -CO-, -CO-O-, -O-CO-, -CO-S-, -S-CO-, -O-CO-O-, -CH=CH-, -C≡C-, Cyclopropyldiyl oder -Si(CH₃)₂- ersetzt sein können, oder R³ und R⁴ können zusammen auch -(CH₂)₄- oder -(CH₂)₅-sein, wenn sie als Substituenten an ein Dioxolan-System gebunden sind;
A¹, A², A³ sind unabhängig voneinander 1,4-Phenylen, Pyrazin-2,5-diyl, Pyridin-2,5-diyl, Pyrimidin-2,5-diyl, bei denen ein oder zwei H-Atome durch F-Atome ersetzt sein können, trans-1,4-Cyclohexylen, (1,3,4)-Thiadiazol-2,5-diyl, Naphthalin-2,6-diyl oder 1,3-Dioxan-2,5-diyl;
A¹ kann auch eine Gruppe der Formel 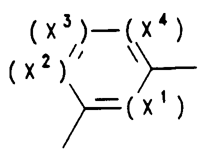 sein;
M¹, M², M³ sind unabhängig voneinander -O-, -CO-O-, -O-CO-, -CH₂-O-, -O-CH₂-, -CH₂-CH₂-, -CH=CH- oder -C≡C-;
M⁴ ist -CH₂-O-, -O-CH₂-, -CO-O-, -O-CO- oder eine Einfachbindung;
X¹, X², X³, X⁴ sind CH, CF oder N, wobei die Zahl der N-Atome 0, 1 oder 2 beträgt.

4. Verbindungen nach Anspruch 1, wobei die Symbole und Indizes folgende Bedeutung haben:
R¹, R² sind unabhängig voneinander ein Alkylrest mit 1 bis 8 C-Atomen, wobei eine oder zwei CH₂-Gruppen durch -O-, -OCO- oder Cyclopropyldiyl ersetzt sein können, oder die chiralen Gruppen: 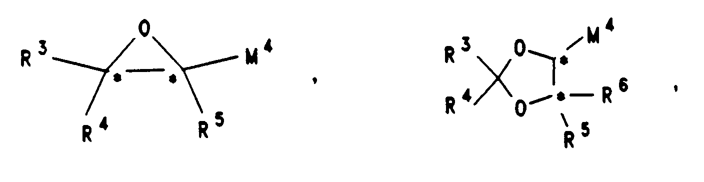
R³, R⁴, R⁵ sind unabhängig voneinander H oder ein geradkettiger oder verzweigter Alkylrest mit 1 bis 8 C-Atomen;
A¹, A², A³ sind unabhängig voneinander 1,4-Phenylen, Pyridin-2,5-diyl, Pyrimidin-2,5-diyl, bei denen ein oder zwei H-Atome durch F-Atome ersetzt sein können oder trans-1,4-Cyclohexylen;
A¹ kann auch eine Gruppe der Formel 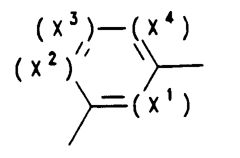 sein;
M¹, M², M³ sind unabhängig voneinander -O-, -CO-O-, -O-CO-, -O-CH₂-, -CH₂-O- oder -CH₂-CH₂-;
M⁴ ist -CH₂-O- oder -CO-O-;
X¹, X², X³, X⁴ sind CH, CF oder N, wobei die Zahl der N-Atome 0, 1 oder 2 beträgt.

5. Flüssigkristallmischung, enthaltend mindestens eine Verbindung nach einem oder mehreren der Ansprüche 1 bis 4.

6. Flüssigkristallmischung nach Anspruch 5, dadurch gekennzeichnet, daß die Flüssigkristallmischung ferroelektrisch ist.

7. Flüssigkristallmischung nach Anspruch 5 oder 6, dadurch gekennzeichnet, daß sie 0,01 bis 60 Gew.-% an mindestens einer Verbindung der Formel (I) enthält.

8. Schalt- und/oder Anzeigevorrichtung, enthaltend Trägerplatten, Elektroden, mindestens einen Polarisator, mindestens eine Orientierungsschicht sowie ein flüssigkristallines Medium, dadurch gekennzeichnet, daß das flüssigkristalline Medium eine Flüssigkristallmischung nach einem oder mehreren der Ansprüche 5 bis 7 ist.

9. Verwendung von Verbindungen der Formel (I) 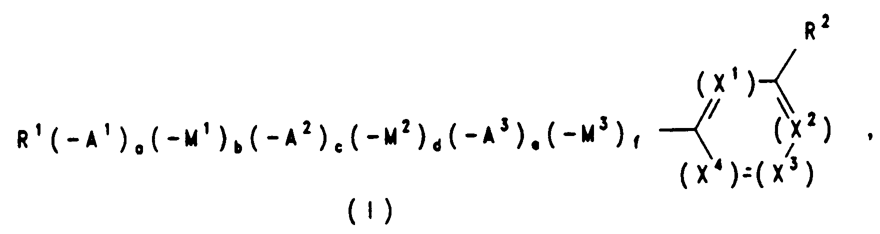 in der die Symbole und Indizes folgende Bedeutung haben:
R¹, R² sind unabhängig voneinander ein geradkettiger oder verzweigter Alkylrest mit 1 bis 22 C-Atomen (mit oder ohne asymmetrische C-Atome), wobei auch eine oder zwei CH₂-Gruppen durch -O-, -S-, -CO-, -CO-O-, -O-CO-, -CO-S-, -S-CO-, -O-CO-O-, -CH=CH-, -C≡C-, Cyclopropyldiyl oder -Si(CH₃)₂- ersetzt sein können und wobei auch ein oder mehrere H-Atome des Alkylrestes durch F, Cl, Br oder CN substituiert sein können, oder eine der nachfolgenden chiralen Gruppen: 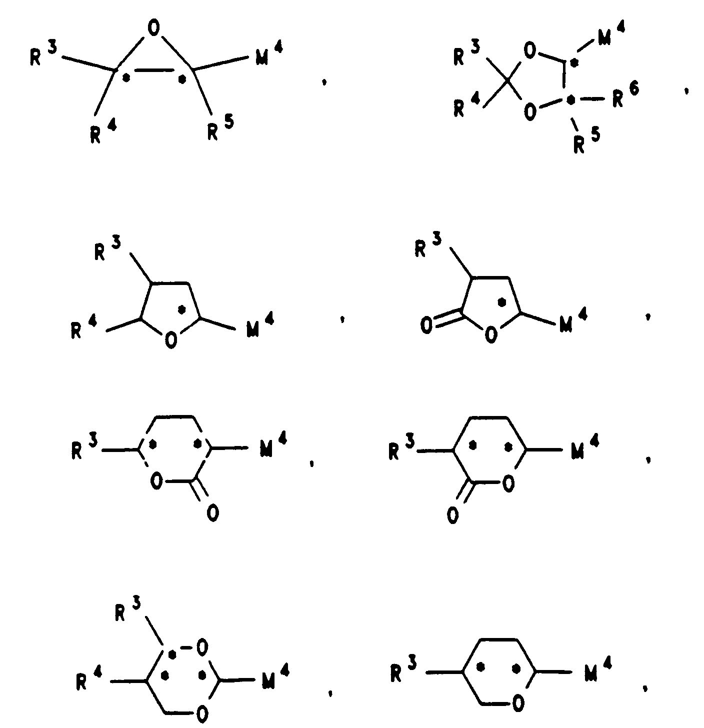 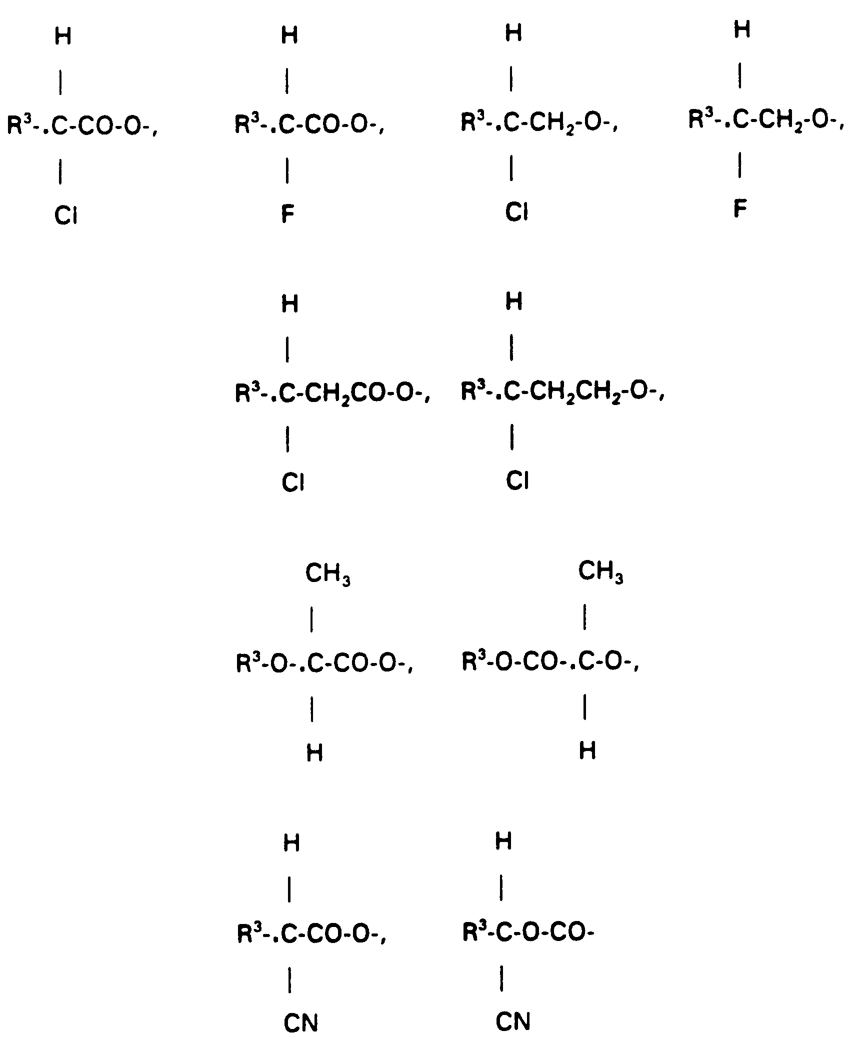
R³, R⁴, R⁵, R⁶ sind unabhängig voneinander H oder ein geradkettiger oder verzweigter Alkylrest mit 1 bis 22 C-Atomen, wobei auch eine oder zwei CH₂-Gruppen durch -O-, -S-, -CO-, -CO-O, -O-CO-, -CO-S-, -S-CO-, -O-CO-O-, -CH=CH-, -C≡C-, Cyclopropyldiyl oder -Si(CH₃)₂- ersetzt sein können, oder R³ und R⁴ können zusammen auch -(CH₂)₄- oder -(CH₂)₅-sein, wenn sie als Substituenten an ein Dioxolan-System gebunden sind;
A¹, A², A³ sind unabhängig voneinander 1,4-Phenylen, wobei ein oder zwei H-Atome durch F, Cl und/oder CN ersetzt sein können, Pyrazin-2,5-diyl, Pyridazin-3,6-diyl, Pyridin-2,5-diyl, Pyrimidin-2,5-diyl, bei denen ein oder zwei H-Atome durch F ersetzt sein können, trans-1,4-Cyclohexylen, bei dem ein oder zwei H-Atome durch -CN und/oder -CH₃ ersetzt sein können, (1,3,4)-Thiadiazol-2,5-diyl, 1,3-Dioxan-2,5-diyl, 1,3-Dithian-2,5-diyl, 1,3-Thiazol-2,4-diyl, 1,3-Thiazol-2,5-diyl, Thiophen-2,4-diyl, Thiophen-2,5-diyl, Piperazin-1,4-diyl, Piperazin-2,5-diyl, Naphthalin-2,6-diyl, Bicyclo[2.2.2]octan-1,4-diyl, 1,3-Dioxaborinan-2,5-diyl oder trans-Dekalin-2,6-diyl;
A¹ kann auch eine Gruppe der Formel 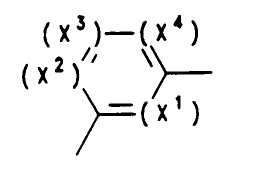 sein;
M¹, M², M³ sind unabhängig voneinander -O-, -S-, -CO-, -CO-O-, -O-CO-, -CO-S-, -S-CO-, -O-CO-O-, -CH₂-O-, -O-CH₂-, -CH₂-CH₂-, -CH=CH- oder -C≡CH-;
M⁴ ist -CH₂-O-, -O-CH₂-, -CO-O-, -O-CO- oder ein Einfachbindung;
X¹, X², X³, X⁴ sind CH, CF oder N, wobei die Zahl der N-Atome 0, 1 oder 2 beträgt;
a, b, c, d, e, f sind null oder eins, unter der Bedingung, daß die Summe a + c + e 1, 2 oder 3 ist;
* ist ein chirales Zentrum,
in Flüssigkristallmischungen zur verbesserung des optischen Schaltwinkels und zur Reduktion der kritischen Pulsfläche beim elektrooptischen Schalten.

## Claims

1. Compounds of the formula (I) 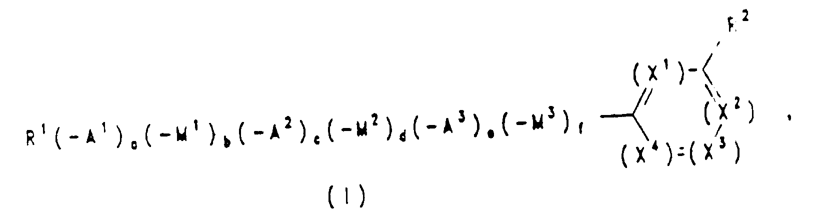 in which the symbols and indices have the following meanings:
R¹ and R², independently of one another, are a straight-chain or branched alkyl radical hiving 1 to 22 carbon atoms (with or without asymmetrical carbon atoms), it also being possible for one or two -CH₂-groups to be replaced by -O-, -S-, -CO-, -CO-O-, -O-CO-, -CO-S-, -S-CO-, -O-CO-O-, -CH=CH-, -C≡C-, cyclopropanediyl or -Si(CH₃)₂-, and it also being possible for one or more hydrogen atoms in the alkyl radical to be substituted by F, Cl, Br or CN, or are one of the following chiral groups: 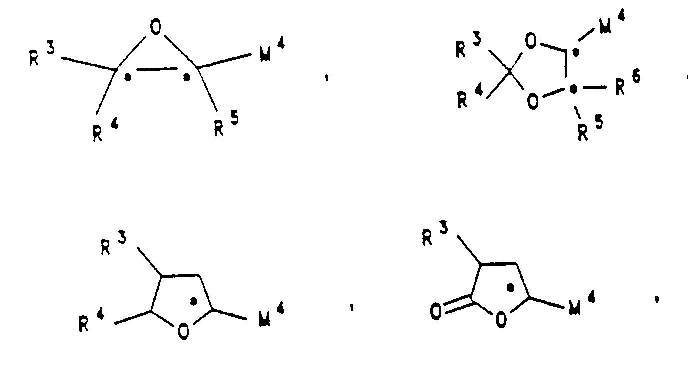 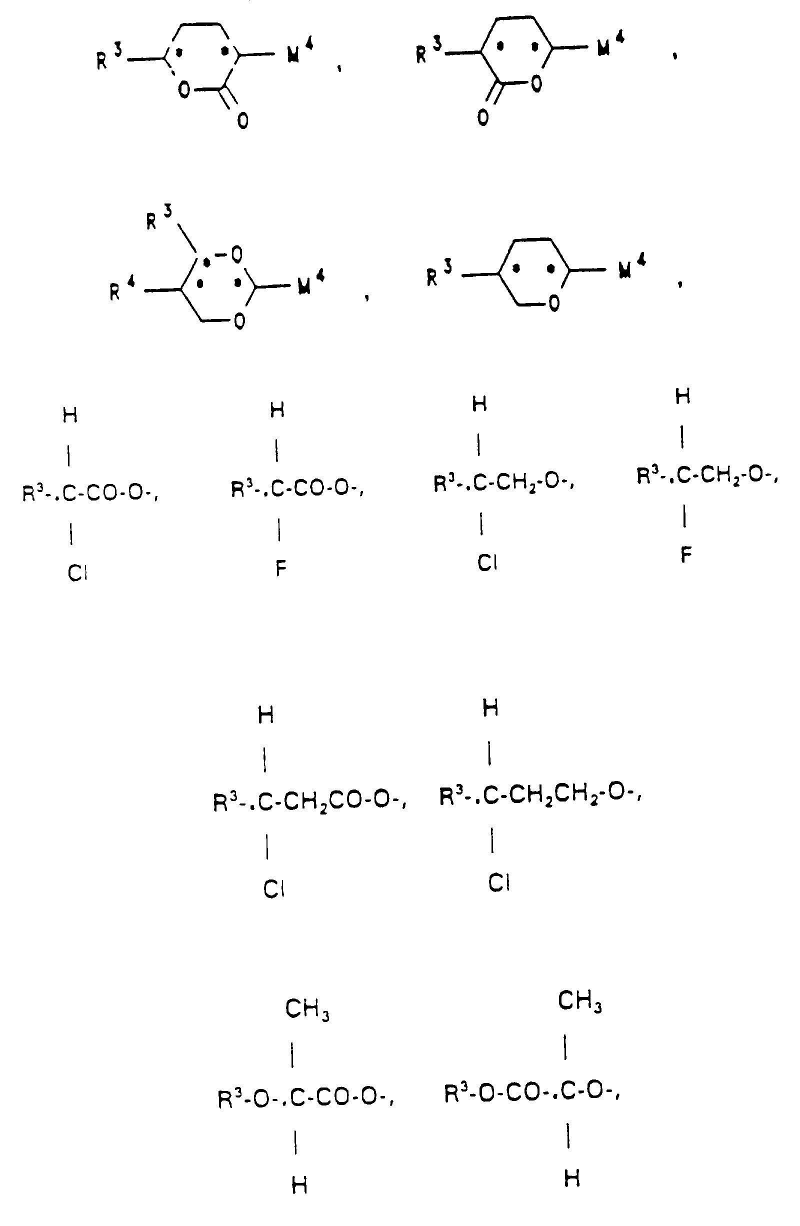 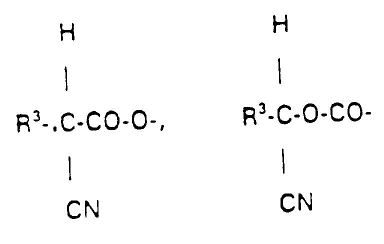
R³, R⁴, R⁵ and R⁶, independently of one another, are H or a straight-chain or branched alkyl radical having 1 to 22 carbon atoms, it also being possible for one or two -CH₂- groups to be replaced by -O-, -S-, -CO-, -CO-O-, -O-CO-, -CO-S-, -S-CO-, -O-CO-O-, -CH=CH-, -C≡C-, cyclopropanediyl or -Si(CH₃)₂-, or R³ and R⁴ together may alternatively be -(CH₂)₄- or -(CH₂)₅- if they are bonded as substituents to a dioxolane system;
A¹, A² and A³, independently of one another, are 1,4-phenylene, in which one or two hydrogen atoms may be replaced by F, Cl and/or CN, pyrazine-2,5-diyl, pyridazine-3,6-diyl, pyridine-2,5-diyl, pyrimidine-2,5-diyl, in which one or two hydrogen atoms may be replaced by F, trans-1,4-cyclohexylene, in which one or two hydrogen atoms may be replaced by -CN and/or -CH₃, 1,3,4-thiadiazole-2,5-diyl, 1,3-dioxane-2,5-diyl, 1,3-dithiane-2,5-diyl, 1,3-thiazole-2,4-diyl, 1,3-thiazole-2,5-diyl, thiophene-2,4-diyl, thiophene-2,5-diyl, piperazine-1,4-diyl, piperazine-2,5-diyl, naphthalene-2,6-diyl, bicyclo[2.2.2]octane-1,4-diyl, 1,3-dioxaborinane-2,5-diyl or trans-decalin-2,6-diyl;
A¹ may alternatively be a group of the formula 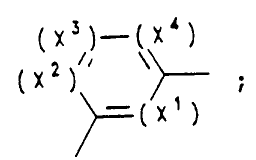
M¹, M² and M³, independently of one another, are -O-, -S-, -CO-, -CO-O-, -O-CO-, -CO-S-, -S-CO-, -O-CO-O-, -CH₂-O-, -O-CH₂-, -CH₂CH₂-, -CH=CH- or -C≡CH-;
M⁴ is -CH₂-O-, -O-CH₂-, -CO-O-, -O-CO- or a single bond;
X¹, X², X³ and X⁴ are CH, CF or N, where the number of nitrogen atoms is 0, 1 or 2;
a, b, c, d, e and f are zero or one, with the proviso that the total a + c + e is 1, 2 or 3;
* is a center of chirality,
compounds with M³: -COO-, -O-CO-, -O-CH₂- or -CH₂O-; f:1; and 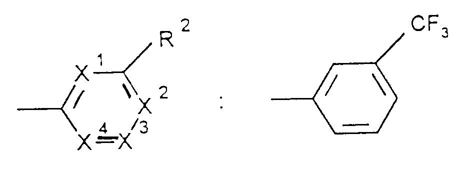 being excluded.

2. Compounds as claimed in claim 1, the symbols and indices having the following meanings: R¹ and R², independently of one another, are a straight-chain or branched alkyl radical having 1 to 15 carbon atoms (with or without asymmetrical carbon atoms), it also being possible for one or two -CH₂-groups to be replaced by -O-, -S-, -CO-, -CO-O-, -O-CO-, -OCO-O-, -CH=CH-, -C≡C-, cyclopropanediyl or -Si(CH₃)₂-, or are one of the following chiral groups: 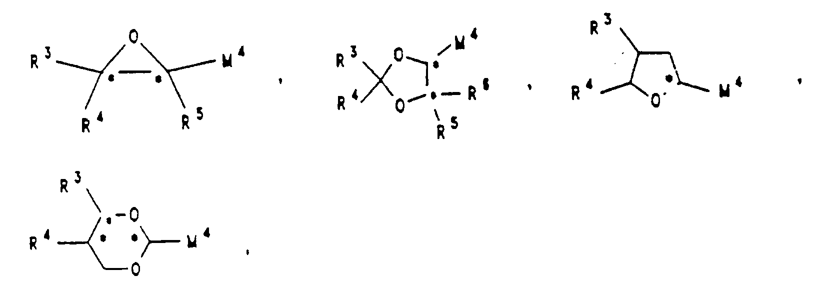 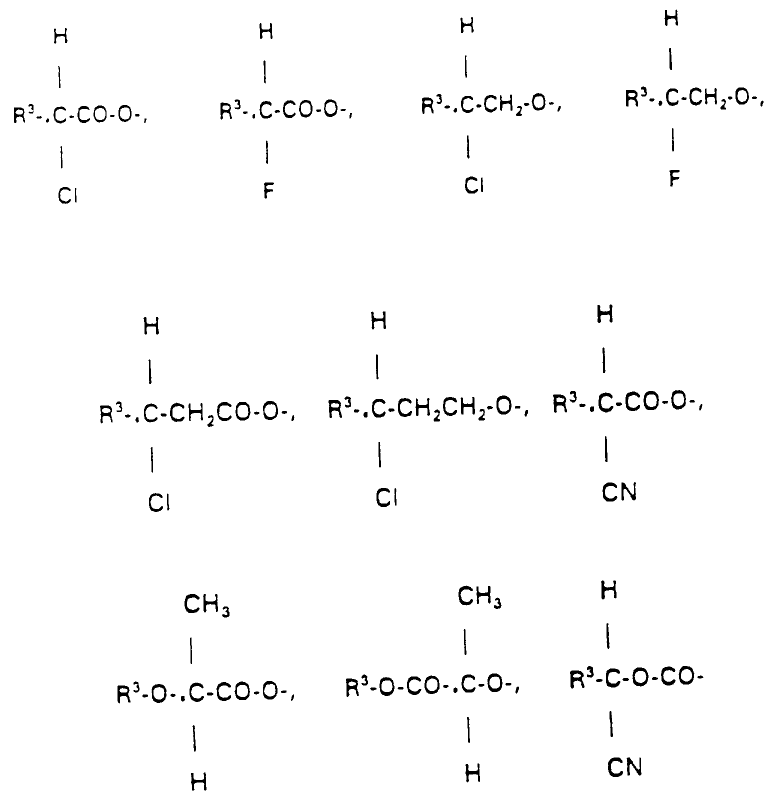
R³, R⁴, R⁵ and R⁶, independently of one another, are H or a straight-chain or branched alkyl radical having 1 to 15 carbon atoms, it also being possible for one or two -CH₂- groups to be replaced by -O-, -S-, -CO-, -CO-O-, -O-CO-, -CO-S-, -S-CO-, -O-CO-O-, -CH=CH-, -C≡C-, cyclopropanediyl or -Si(CH₃)₂-, or R³ and R⁴ together may alternatively be -(CH₂)₄- or -(CH₂)₅- if they are bonded as substituents to a dioxolane system;
A¹, A² and A³, independently of one another, are 1,4-phenylene, pyrazine-2,5-diyl, pyridazine-3,6-diyl, pyridine-2,5-diyl, pyrimidine-2,5-diyl, in which one or two hydrogen atoms may be replaced by F, trans-1,4-cyclohexylene, 1,3,4-thiadiazole-2,5-diyl, 1,3-dioxane-2,5-diyl, naphthalene-2,6-diyl or bicyclo[2.2.2]octanel-1,4-diyl;
A¹ may alternatively be a group of the formula 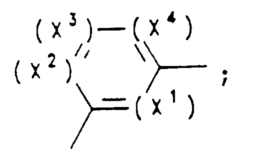
M¹, M² and M³, independently of one another, are -O-, -CO-, -CO-O-, -O-CO-, -CH₂-O-, -O-CH₂-, -CH₂CH₂-, -CH=CH- or -C≡C-;
M⁴ is -CH₂-O-, -O-CH₂-, -CO-O-, -O-CO- or a single bond;
X¹, X², X³ and X⁴ are CH, CF or N, where the number of nitrogen atoms is 0, 1 or 2.

3. Compounds as claimed in claim 1, the symbols and indices having the following meanings: R¹ and R², independently of one another, are a straight-chain or branched alkyl radical having 1 to 10 carbon atoms (with or without asymmetrical carbon atoms), it also being possible for one or two -CH₂-groups to be replaced by -O-, -CO-, -CO-O-, -O-CO-, -CH=CH-, -C≡C-, cyclopropanediyl or -Si(CH₃)₂-, or are one of the following chiral groups: 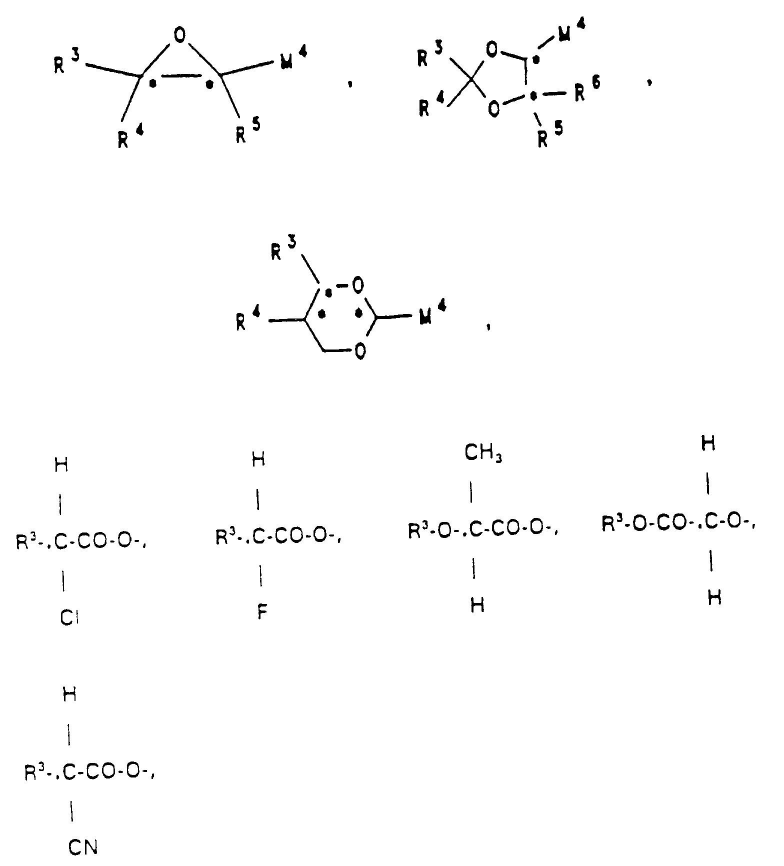
R³, R⁴, R⁵ and R⁶, independently of one another, are H or a straight-chain or branched alkyl radical having 1 to 10 carbon atoms, it also being possible for one or two -CH₂- groups to be replaced by -O-, -S-, -CO-, -CO-O-, -O-CO-, -CO-S-, -S-CO-, -O-CO-O-, -CH=CH-, -C≡C-, cyclopropanediyl or -Si(CH₃)₂-, or R³ and R⁴ together may alternatively be -(CH₂)₄- or -(CH₂)₅- if they are bonded as substituents to a dioxolane system;
A¹, A² and A³, independently of one another, are 1,4-phenylene, pyrazine-2,5-diyl, pyridine-2,5-diyl, pyrimidine-2,5-diyl, in which one or two hydrogen atoms may be replaced by F atoms, trans-1,4-cyclohexylene, 1,3,4-thiadiazole-2,5-diyl, naphthalene-2,6-diyl or 1,3-dioxane-2,5-diyl;
A¹ may alternatively be a group of the formula 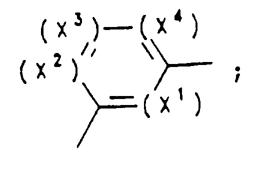
M¹, M² and M³, independently of one another, are -O-, -CO-O-, -O-CO-, -CH₂-O-, -O-CH₂-, -CH₂CH₂-, -CH=CH-or -C≡C-;
M⁴ is -CH₂-O-, -O-CH₂-, -CO-O-, -O-CO- or a single bond;
X¹, X², X³ and X⁴ are CH, CF or N, where the number of nitrogen atoms is 0, 1 or 2.

4. Compounds as claimed in claim 1, the symbols and indices having the following meanings: R¹ and R², independently of one another, are an alkyl radical having 1 to 8 carbon atoms, it being possible for one or two -CH₂- groups to be replaced by -O-, -OCO- or cyclopropanediyl, or are the chiral groups: 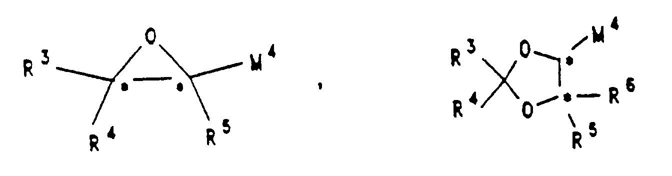
R³, R⁴ and R⁵, independently of one another, are H or a straight-chain or branched alkyl radical having 1 to 8 carbon atoms;
A¹, A² and A³, independently of one another, are 1,4-phenylene, pyridine-2,5-diyl, pyrimidine-2,5-diyl, in which one or two hydrogen atoms may be replaced by F atoms, or trans-1,4-cyclohexylene;
A¹ may alternatively be a group of the formula 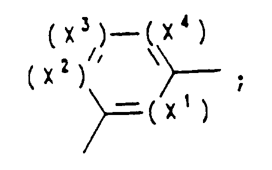
M¹, M² and M³, independently of one another, are -O-, -CO-O-, -O-CO-, -O-CH₂-, -CH₂-O- or -CH₂CH₂-;
M⁴ is -CH₂-O- or -CO-O-;
X¹, X², X³ and X⁴ are CH, CF or N, where the number of nitrogen atoms is 0, 1 or 2.

5. A liquid-crystal mixture containing at least one compound as claimed in one or more of claims 1 to 4.

6. A liquid-crystal mixture as claimed in claim 5, wherein the liquid-crystal mixture is ferroelectric.

7. A liquid-crystal mixture as claimed in claim 5 or 6, which contains from 0.01 to 60% by weight of at least one compound of the formula (I).

8. A switching and/or display device comprising outer plates, electrodes, at least one polarizer, at least one alignment layer and a liquid-crystalline medium, wherein the liquid-crystalline medium is a liquid-crystal mixture as claimed in one or more of claims 5 to 7.

9. The use of compounds of the formula (I) 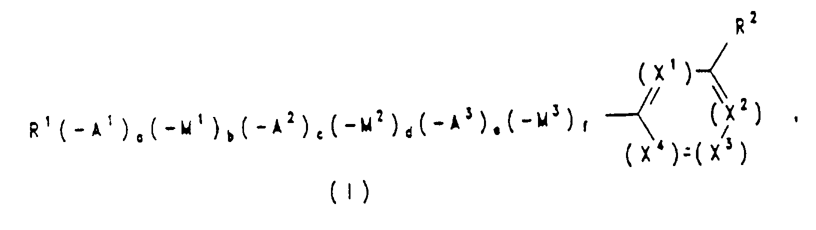 in which the symbols and indices have the following meanings:
R¹ and R², independently of one another, are a straight-chain or branched alkyl radical having 1 to 22 carbon atoms (with or without asymmetrical carbon atoms), it also being possible for one or two -CH₂-groups to be replaced by -O-, -S-, -CO-, -CO-O-, -O-CO-, -CO-S-, -S-CO-, -O-CO-O-, -CH=CH-, -C≡C-, cyclopropanediyl or -Si(CH₃)₂-, and it also being possible for one or more hydrogen atoms in the alkyl radical to be substituted by F, Cl, Br or CN, or are one of the following chiral groups: 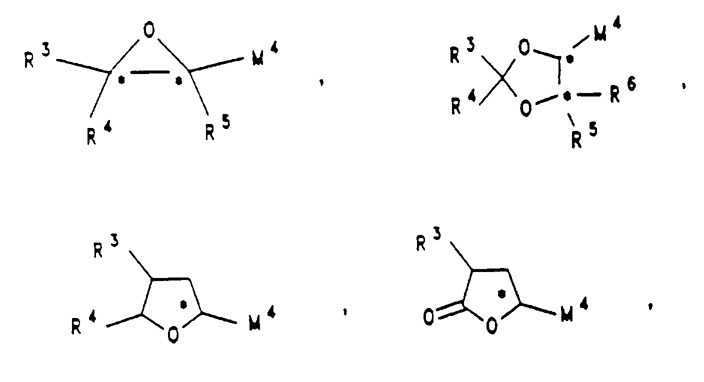
M¹, M² and M³, independently of one another, are -O-, -S-, -CO-, -CO-O-, -O-CO-, -CO-S-, -S-CO-, -O-CO-O-, -CH₂-O-, -O-CH₂-, -CH₂CH₂-, -CH=CH- or -C≡CH-;
M⁴ is -CH₂-O-, -O-CH₂-, -CO-O-, -O-CO- or a single bond;
X¹, X², X³ and X⁴ are CH, CF or N, where the number of nitrogen atoms is 0, 1 or 2;
a, b, c, d, e and f are zero or one, with the proviso that the total a + c + e is 1, 2 or 3;
* is a center of chirality,
in liquid crystal mixtures for improving the optical switch angle and for reducing the critical pulse area during electro-optical switching.

## Revendications

1. Composés de formule (I) 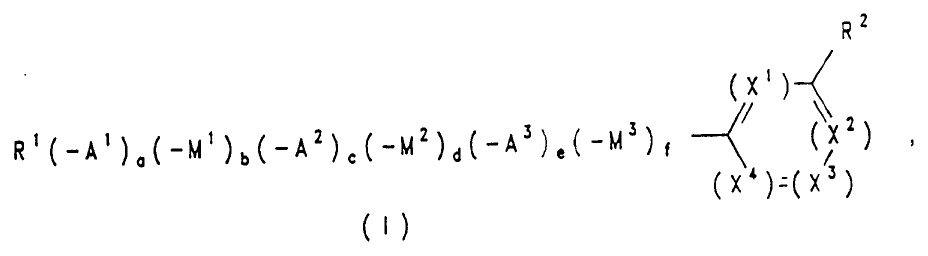 dans laquelle les symboles et indices ont la signification suivante :
R¹, R² sont indépendamment l'un de l'autre un groupe alkyle à chaîne linéaire ou ramifiée avec 1 à 22 atomes de carbone (avec ou sans atomes de carbone asymétriques), dans lequel un ou deux groupes CH₂ peuvent être remplacés par -O-, -S-, -CO-, -CO-O-, -O-CO-, -CO-S-, -S-CO-, -O-CO-O-, -CH=CH-, -C≡C-, le groupe cyclopropyldiyle ou -Si(CH₃)₂- et dans lequel également un ou plusieurs atomes d'hydrogène du groupe alkyle peuvent être substitués par F, Cl, Br ou CN, ou un des groupes chiraux suivants : 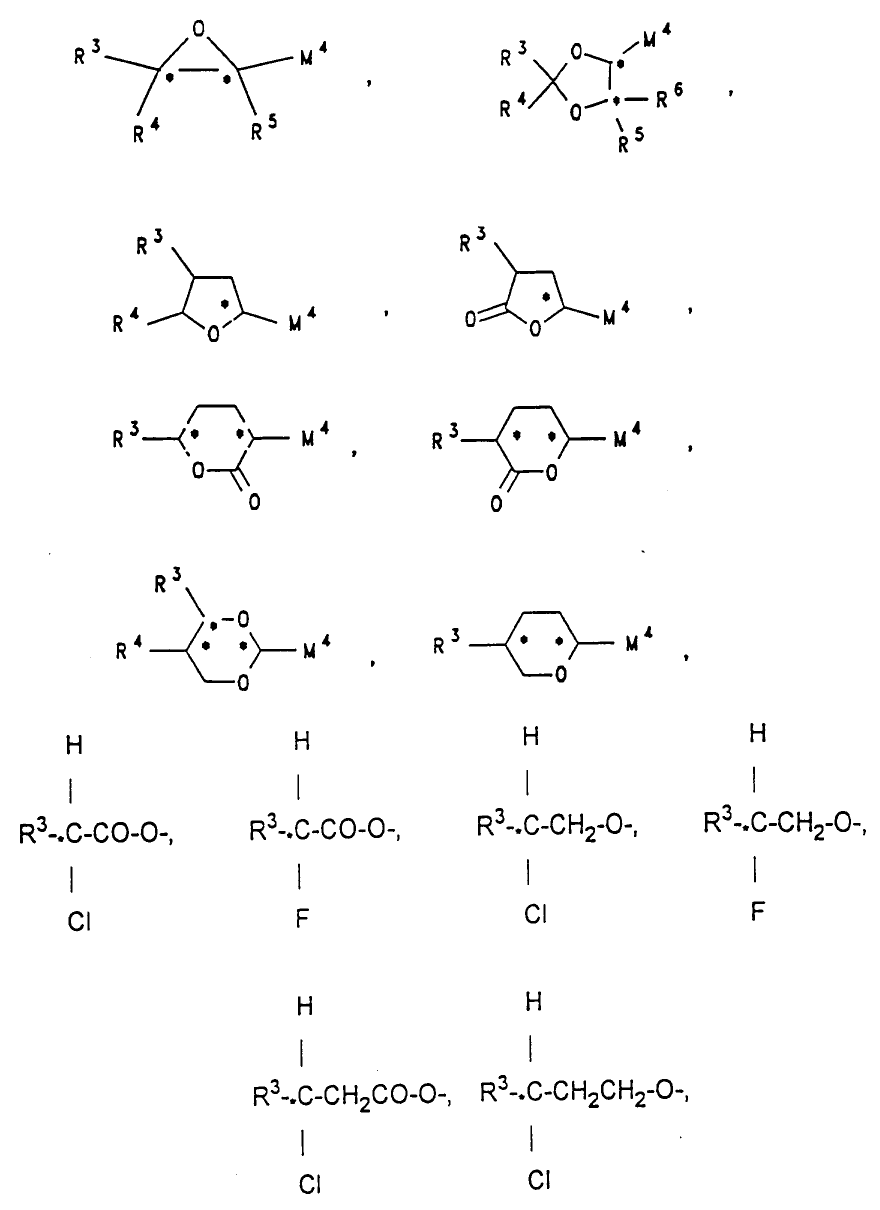 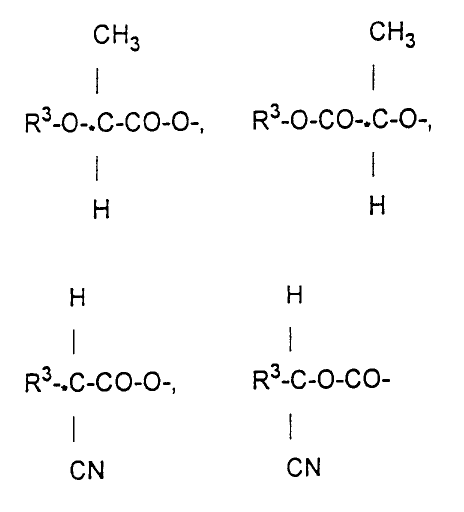
R³, R⁴, R⁵, R⁶ sont indépendamment les uns des autres H ou un groupe alkyle à chaîne linéaire ou ramifiée avec 1 à 22 atomes de carbone, dans lequel également un ou deux groupes CH₂ peuvent être remplacés par -O-, -S-, -CO-, - CO-O-, -O-CO-, -CO-S-, -S-CO-, -O-CO-O-, -CH=CH-, -C≡C-, le groupe cyclopropyldiyle ou -Si(CH₃)₂-, ou R³ et R⁴ peuvent aussi représenter ensemble -(CH₂)₄- ou -(CH₂)₅-, lorsqu'ils sont liés comme substituants à un système dioxolane;
A¹, A², A³ sont indépendamment les uns des autres un groupe 1,4-phénylène, dans lequel un ou deux atomes d'hydrogène peuvent être remplacés par F, Cl et/ou CN, le groupe pyrazin-2,5-diyle, pyrazin-3,6-diyle, pyridin-2,5-diyle, pyrimidin-2,5-diyle, dans lesquels un ou deux atomes d'hydrogène peuvent être remplacés par F, trans-1,4-cyclohexylène, dans lequel un ou deux atomes d'hydrogène peuvent être remplacés par -CN et/ou -CH₃, (1,3,4)-thiadiazol-2,5-diyle, 1,3-dioxan-2,5-diyle, 1,3-dithian-2,5-diyle, 1,3-thiazol-2,4-diyle, 1,3-thiazol-2,5-diyle, thiophèn-2,4-diyle, thiophèn-2,5-diyle, pipérazin-1,4-diyle, pipérazin-2,5-diyle, naphtalèn-2,6-diyle, bicyclo[2.2.2]octan-1,4-diyle, 1,3-dioxaborinan-2,5-diyle ou trans-décalin-2,6-diyle;
A¹ peut aussi être un groupe de formule 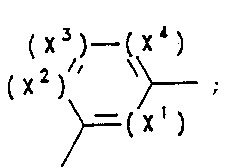
M¹, M², M³ sont indépendamment les uns des autres -O-, -S-, -CO-, -CO-O-, -O-CO, -CO-S-, -S-CO-, -O-CO-O-, -CH₂-O-, -O-CH₂-, -CH₂-CH₂-, -CH=CH- ou -C≡C-;
M⁴ est -CH₂-O-, -O-CH₂-, -CO-O-, -O-CO- ou une simple liaison;
X¹, X², X³, X⁴ sont CH, CF ou N, dans lesquels le nombre des atomes d'azote s'élève à 0, 1 ou 2;
a, b, c, d, e, f sont zéro ou un, à condition que la somme a +c + e soit de 1, 2 ou 3;
* représente un centre chiral,
dans lesquels les composés avec
M³ : -CO-O-, -O-CO-, -O-CH₂- ou -CH₂-O-;
f : 1; et 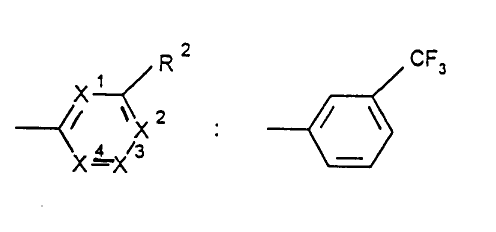 sont exclus.

2. Composés selon la revendication 1, dans lesquels les symboles et indices ont la signification suivante :
R¹, R² sont indépendamment l'un de l'autre un groupe alkyle à chaîne linéaire ou ramifiée avec 1 à 15 atomes de carbone (avec ou sans atomes de carbone asymétriques), dans lequel un ou deux groupes CH₂ peuvent être remplacés par -O-, -S-, -CO-, -CO-O-, -O-CO-, -O-CO-O-, -CH=CH-, -C≡C-, le groupe cyclopropyldiyle ou -Si(CH₃)₂- ou un des groupes chiraux suivants : 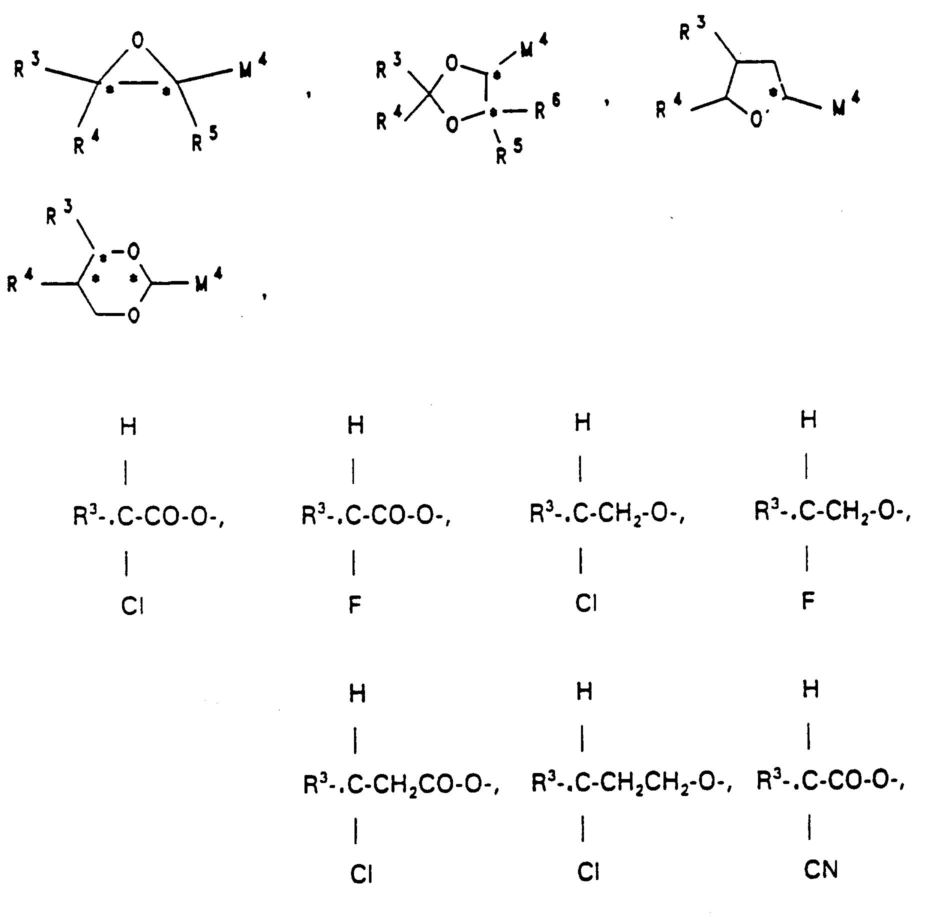 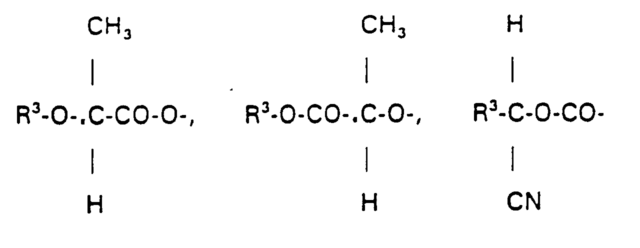
R³, R⁴, R⁵, R⁶ sont indépendamment les uns des autres H ou un groupe alkyle à chaîne linéaire ou ramifiée avec 1 à 15 atomes de carbone, dans lequel également un ou deux groupes CH₂ peuvent être remplacés par -O-, -S-, -CO-, -CO-O-, -O-CO-, -CO-S-, -S-CO-, -O-CO-O-, -CH=CH-, -C≡C-, le groupe cyclopropyldiyle ou -Si(CH₃)₂-, ou R³ et R⁴ peuvent aussi représenter ensemble -(CH₂)₄- ou -(CH₂)₅-, lorsqu'ils sont liés comme substituants à un système dioxolane;
A¹, A², A³ sont indépendamment les uns des autres un groupe 1,4-phénylène, pyrazin-2,5-diyle, pyrazin-3,6-diyle, pyridin-2,5-diyle, pyrimidin-2,5-diyle, dans lesquels un ou deux atomes d'hydrogène peuvent être remplacés par F, trans-1,4-cyclohexylène, (1,3,4)-thiadiazol-2,5-diyle, 1,3-dioxan-2,5-diyle, naphtalèn-2,6-diyle ou bicyclo[2.2.2]octan-1,4-diyle;
A¹ peut aussi être un groupe de formule 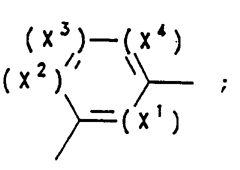
M¹, M², M³ sont indépendamment les uns des autres -O-, -CO-, -CO-O-, -O-CO-, -CH₂-O-, -O-CH₂-, -CH₂-CH₂-, -CH=CH-ou -C≡C-;
M⁴ est -CH₂-O-, -O-CH₂-, -CO-O-, -O-CO- ou une simple liaison;
X¹, X², X³, X⁴ sont CH, CF ou N, dans lesquels le nombre des atomes d'azote s'élève à 0, 1 ou 2.

3. Composé selon la revendication 1, dans lequel les symboles et indices ont la signification suivante : R¹, R² sont indépendamment l'un de l'autre un groupe alkyle à chaîne linéaire ou ramifiée avec 1 à 10 atomes de carbone (avec ou sans atomes de carbone asymétriques), dans lequel un ou deux groupes CH₂ peuvent être remplacés par -O-, -CO-, -CO-O-, -O-CO-, -CH=CH-, -C≡C-, le groupe cyclopropyldiyle ou -Si(CH₃)₂- ou un des groupes chiraux suivants : 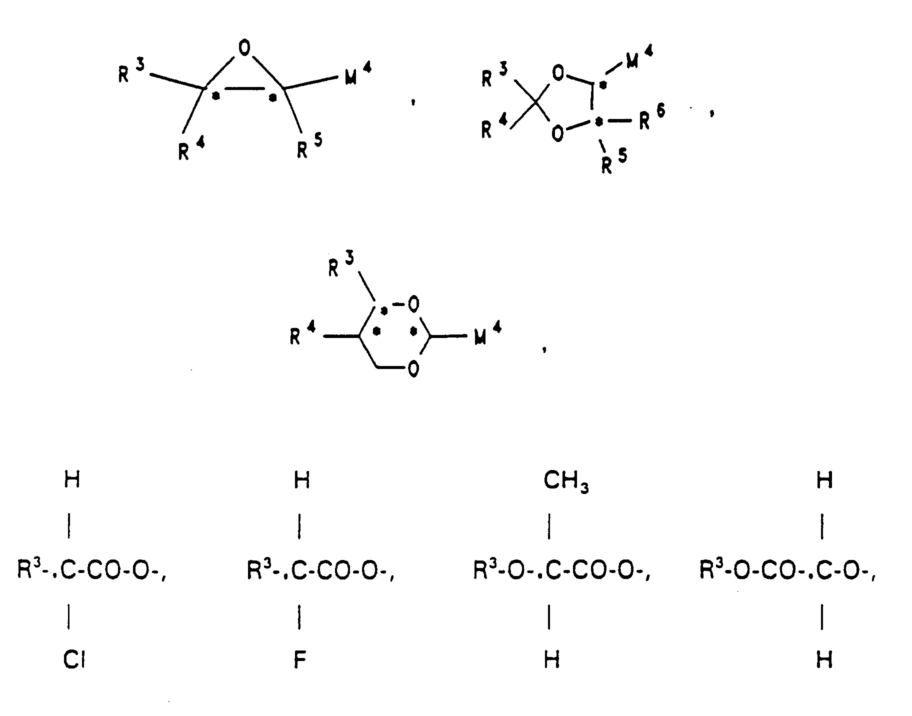 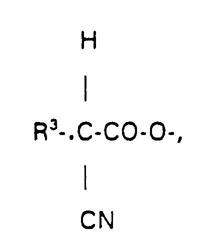
R³, R⁴, R⁵, R⁶ sont indépendamment les uns des autres H ou un groupe alkyle à chaîne linéaire ou ramifiée avec 1 à 10 atomes de carbone, dans lequel également un ou deux groupes CH₂ peuvent être remplacés par -O-, -S-, -CO-, -CO-O-, -O-CO-, -CO-S-, -S-CO-, -O-CO-O-, -CH=CH-, -C≡C-, le groupe cyclopropyldiyle ou -Si(CH₃)₂-, ou R³ et R⁴ peuvent aussi représenter ensemble -(CH₂)₄- ou -(CH₂)₅-, lorsqu'ils sont liés comme substituants à un système dioxolane;
A¹, A², A³ sont indépendamment les uns des autres un groupe 1,4-phénylène, pyrazin-2,5-diyle, pyridin-2,5-diyle, pyrimidin-2,5-diyle, dans lesquels un ou deux atomes d'hydrogène peuvent être remplacés par F, trans-1,4-cyclohexylène, (1,3,4)-thiadiazol-2,5-diyle, naphtalèn-2,6-diyle ou 1,3-dioxan-2,5-diyle;
A¹ peut aussi être un groupe de formule 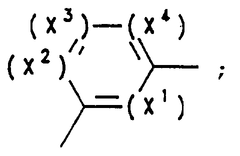
M¹, M², M³ sont indépendamment les uns des autres -O-, -CO-O-, -O-CH₂-, -CH₂-CH₂-, -CH=CH- ou -C≡C-;
M⁴ est -CH₂-O-, -O-CH₂-, -CO-O-, -O-CO- ou une simple liaison;
X¹, X², X³, X⁴ sont CH, CF ou N, dans lesquels le nombre des atomes N s'élève à 0, 1 ou 2.

4. Composés selon la revendication 1, dans lesquels les symboles et indices ont la signification suivante :
R¹, R² sont indépendamment l'un de l'autre un groupe alkyle à chaîne linéaire ou ramifiée avec 1 à 8 atomes de carbone, dans lequel un ou deux groupes CH₂ peuvent être remplacés par -O-, -O-CO- ou le groupe cyclopropyldiyle ou -Si(CH₃)₂- ou les groupes chiraux : 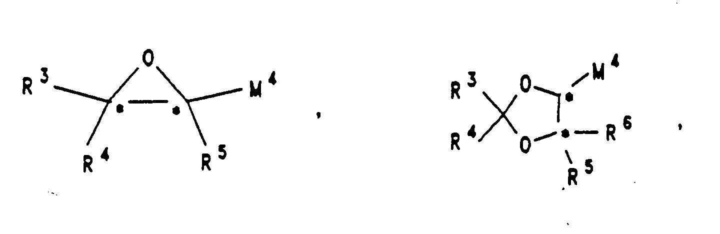
R³, R⁴, R⁵ sont indépendamment les uns des autres H ou un groupe alkyle à chaîne linéaire ou ramifiée avec 1 à 8 atomes de carbone;
A¹, A², A³ sont indépendamment les uns des autres un groupe 1,4-phénylène, pyridin-2,5-diyle, pyrimidin-2,5-diyle, dans lesquels un ou deux atomes d'hydrogène peuvent être remplacés par des atomes F ou le trans-1,4-cyclohexylène;
A¹ peut aussi être un groupe de formule 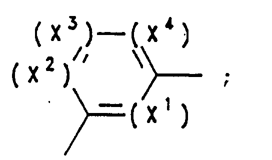
M¹, M², M³ sont indépendamment les uns des autres -O-, -CO-O-, -O-CO-, -O-CH₂-, -CH₂-O- ou -CH₂-CH₂-;
M⁴ est -CH₂-O- ou -CO-O-;
X¹, X², X³, X⁴ sont CH, CF ou N, dans lesquels le nombre des atomes N s'élève à 0, 1 ou 2.

5. Composition de cristal liquide, comprenant au moins un composé selon une ou plusieurs des revendications 1 à 4.

6. Composition de cristal liquide selon la revendication 5, caractérisée en ce que la composition de cristal liquide est ferroélectrique.

7. Composition de cristal liquide selon la revendication 5 ou 6, caractérisée en ce qu'elle contient 0,01 à 60% en poids d'au moins un composé de formule (I).

8. Dispositif de commutation et/ou de signalisation, contenant des plaques de base, des électrodes, au moins un polariseur, au moins une couche d'orientation et un milieu cristal liquide, caractérisé en ce que, le milieu cristal liquide est une composition selon une ou plusieurs des revendications 5 à 7.

9. Utilisation de composés de formule (I) 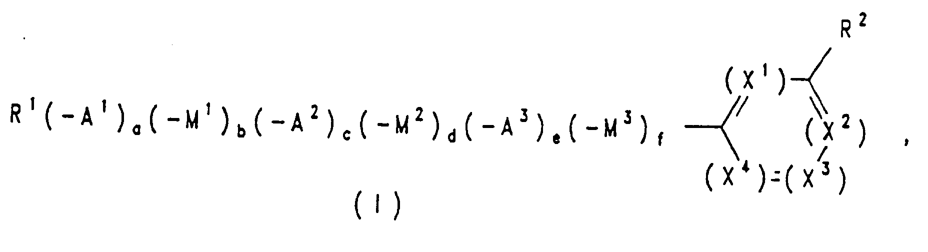 dans laquelle les symboles et indices ont la signification suivante :
R¹, R² sont indépendamment l'un de l'autre un groupe alkyle à chaîne linéaire ou ramifiée avec 1 à 22 atomes de carbone (avec ou sans atomes de carbone asymétriques), dans lequel un ou deux groupes CH₂ peuvent être remplacés par -O-, -S-, -CO-, -CO-O-, -O-CO-, -CO-S-, -S-CO-, -O-CO-O-, -CH=CH-, -C≡C-, le groupe cyclopropyldiyle ou -Si(CH₃)₂- et dans lequel également un ou plusieurs atomes d'hydrogène du groupe alkyle peuvent être substitués par F, Cl, Br ou CN, ou un des groupes chiraux suivants : 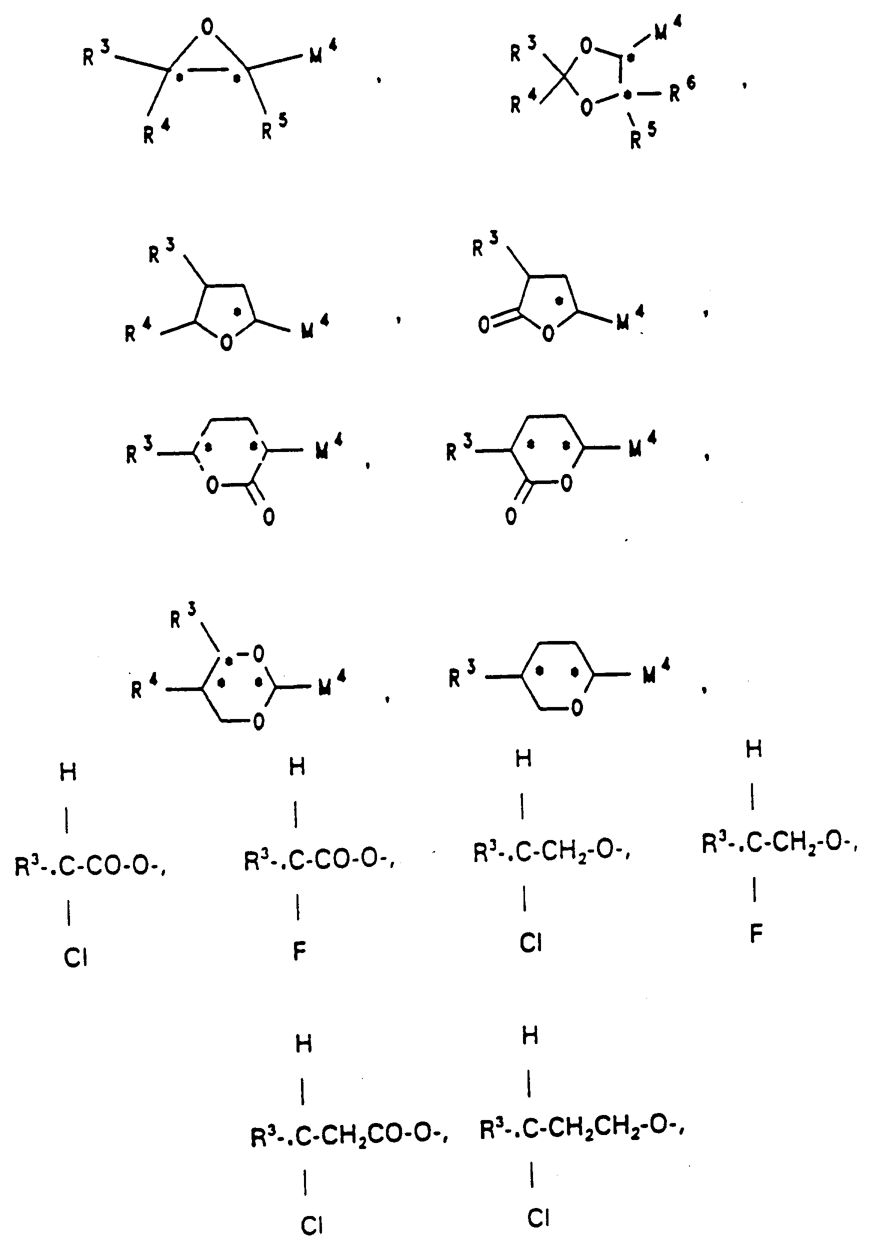 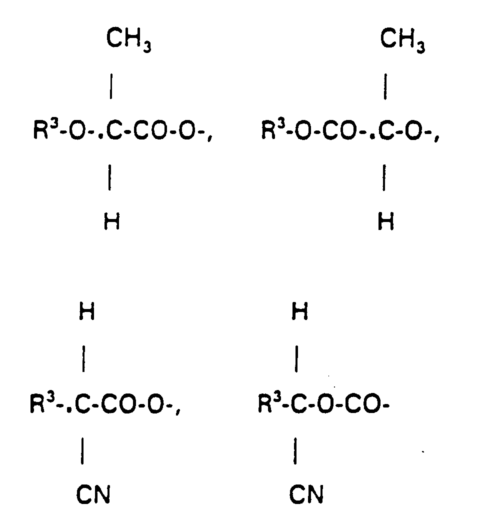
R³, R⁴, R⁵, R⁶ sont indépendamment les uns des autres H ou un groupe alkyle à chaîne linéaire ou ramifiée avec 1 à 22 atomes de carbone, dans lequel également un ou deux groupes CH₂ peuvent être remplacés par -O-, -S-, -CO-, -CO-O-, -O-CO-, -CO-S-, -S-CO-, -O-CO-O-, -CH=CH-, -C≡C-, le groupe cyclopropyldiyle ou -Si(CH₃)₂-, ou R³ et R⁴ peuvent aussi représenter ensemble -(CH₂)₄- ou -(CH₂)₅-, lorsqu'ils sont liés comme substituants à un système dioxolane;
A¹, A², A³ sont indépendamment les uns des autres un groupe 1,4-phénylène, dans lequel un ou deux atomes d'hydrogène peuvent être remplacés par F, Cl et/ou CN, le groupe pyrazin-2,5-diyle, pyrazin-3,6-diyle, pyridin-2,5-diyle, pyrimidin-2,5-diyle, dans lesquels un ou deux atomes d'hydrogène peuvent être remplacés par F, trans-1,4-cyclohexylène, dans lequel un ou deux atomes d'hydrogène peuvent être remplacés par -CN et/ou -CH₃, (1,3,4)-thiadiazol-2,5-diyle, 1,3-dioxan-2,5-diyle, 1,3-dithian-2,5-diyle, 1,3-thiazol-2,4-diyle, 1,3-thiazol-2,5-diyle, thiophèn-2,4-diyle, thiophèn-2,5-diyle, pipérazin-1,4-diyle, pipérazin-2,5-diyle, naphtalèn-2,6-diyle, bicyclo[2.2.2]octan-1,4-diyle, 1,3-dioxaborinan-2,5-diyle ou trans-décalin-2,6-diyle;
A¹ peut aussi être un groupe de formule 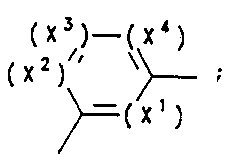
M¹, M², M³ sont indépendamment les uns des autres -O-, -S-, -CO-, -CO-O-, -O-CO-, -CO-S-, -S-CO-, -O-CO-O-, -CH₂-O-, -O-CH₂-, -CH₂-CH₂-, -CH=CH- ou -C≡C-;
M⁴ est -CH₂-O-, -O-CH₂-, -CO-O-, -O-CO- ou une simple liaison;
X¹, X², X³, X⁴ sont CH, CF ou N, dans lesquels le nombre des atomes d'azote s'élève à 0, 1 ou 2;
a, b, c, d, e, f sont zéro ou un, à condition que la somme a +c + e soit de 1, 2 ou 3;
* représente un centre chiral,
dans des compositions de cristal liquide pour améliorer l'angle de commutation optique et réduire la largeur critique d'impulsion dans la commutation électrooptique.
